# EUROPEAN PATENT SPECIFICATION

(11) **EP 2 977 760 B1**
(45) Date of publication and mention of the grant of the patent: **30.05.2018**
(21) Application number: 14767707.4
(22) Date of filing: 17.03.2014
(51) Int. Cl.: G01N 33/574, G01N 33/53, G01N 33/48, G01N 33/50

(54) **BIOMARKER FOR DIAGNOSING LIVER CANCER**
BIOMARKER ZUR DIAGNOSE VON LEBERKREBS
BIOMARQUEUR POUR LE DIAGNOSTIC DU CANCER DU FOIE

(30) Priority: 20.03.2013 KR 20130029626
(43) Date of publication of application: 27.01.2016
(73) Proprietor: Seoul National University R&DB Foundation, Gwanak-gu Seoul 08826 (KR)
(72) Inventor: KIM, Youngsoo, Seoul 135-280 (KR); YOON, Junghwan, Seoul 110-744 (KR); KIM, Hyunsoo, Bucheon-si Gyeonggi-do 420-804 (KR); JIN, Jonghwa, Seoul 136-725 (KR)
(74) Representative: Ter Meer Steinmeister & Partner
(86) International application number: PCT/KR2014/002209
(87) International publication number: WO 2014/148780

(56) References cited:
- EP-B1- 2 126 121
- WO-A2-2009/105154
- JP-A- 2006 308 533
- JP-A- 2009 034 092
- KR-A- 20120 021 518
- US-A1- 2003 087 316
- TARA BEHNE ET AL: "Biomarkers for Hepatocellular Carcinoma", INTERNATIONAL JOURNAL OF HEPATOLOGY, vol. 2012, no. 7, 1 January 2012 (2012-01-01), pages 1-7, XP055298818, US ISSN: 2090-3448, DOI: 10.1155/2012/859076
- SEON-HEE YIM ET AL: "An Overview of Biomarkers and Molecular Signatures in HCC", CANCERS, vol. 2, no. 2, 7 May 2010 (2010-05-07), pages 809-823, XP055049053, DOI: 10.3390/cancers2020809
- BOYAULT SANDRINE ET AL: "Transcriptome classification of HCC is related to gene alterations and to new therapeutic targets", HEPATOLOGY,, vol. 45, no. 1, 1 January 2007 (2007-01-01), pages 42-52, XP002598559, ISSN: 0270-9139, DOI: 10.1002/HEP.21467 [retrieved on 2006-12-22]
- COSTANTINI S ET AL: "Gene expression signature of human HepG2 cell line", GENE, ELSEVIER, AMSTERDAM, NL, vol. 518, no. 2, 26 January 2013 (2013-01-26), pages 335-345, XP028988871, ISSN: 0378-1119, DOI: 10.1016/J.GENE.2012.12.106
- CHIA-CHU TSAI ET AL: "Gene expression analysis of human hepatocellular carcinoma by using full-length cDNA library", JOURNAL OF BIOMEDICAL SCIENCE, KLUWER ACADEMIC PUBLISHERS, DO, vol. 13, no. 2, 1 March 2006 (2006-03-01), pages 241-249, XP019272268, ISSN: 1423-0127
- OKABE H ET AL: "Genome-wide analysis of gene expression in human hepatocellular carcinomas using cDNA microarray: Identification of genes involved in viral carcinogenesis and tumor progression", CANCER RESEARCH, AMERICAN ASSOCIATION FOR CANCER RESEARCH, US, vol. 61, no. 5, 1 March 2001 (2001-03-01), pages 2129-2137, XP002235524, ISSN: 0008-5472
- ATSUSHI YANAGITANI ET AL: "Retinoic acid receptor ? dominant negative form causes steatohepatitis and liver tumors in transgenic mice", HEPATOLOGY, vol. 40, no. 2, 1 August 2004 (2004-08-01) , pages 366-375, XP055298794, ISSN: 0270-9139, DOI: 10.1002/hep.20335
- RAGHOTHAMA CHAERKADY ET AL: "A Quantitative Proteomic Approach for Identification of Potential Biomarkers in Hepatocellular Carcinoma", JOURNAL OF PROTEOME RESEARCH, vol. 7, no. 10, 3 October 2008 (2008-10-03), pages 4289-4298, XP055007768, ISSN: 1535-3893, DOI: 10.1021/pr800197z
- LANGHO LEE ET AL: "Liverome: a curated database of liver cancer-related gene signatures with self-contained context information", BMC GENOMICS, vol. 12, no. Suppl 3, 1 January 2011 (2011-01-01), page S3, XP055299013, London, UK ISSN: 1471-2164, DOI: 10.1371/journal.pone.0020090
- S. TANAKA ET AL: "Aurora kinase B is a predictive factor for the aggressive recurrence of hepatocellular carcinoma after curative hepatectomy", BRITISH JOURNAL OF SURGERY., vol. 95, no. 5, 1 May 2008 (2008-05-01), pages 611-619, XP055326745, GB ISSN: 0007-1323, DOI: 10.1002/bjs.6011
- CHIANG DEREK Y ET AL: "Focal gains of VEGFA and molecular classification of hepatocellular carcinoma", CANCER RESEARCH, AMERICAN ASSOCIATION FOR CANCER RESEARCH, US, vol. 68, no. 16, 15 August 2008 (2008-08-15), pages 6779-6788, XP002598557, ISSN: 0008-5472, DOI: 10.1158/0008-5472.CAN-08-0742
- YE QING-HAI ET AL: "Predicting hepatitis B virus-positive metastatic hepatocellular carcinomas using gene expression profiling and supervised machine learning", NATURE MEDICINE, NATURE PUBLISHING GROUP, NEW YORK, NY, US, vol. 9, no. 4, 1 April 2003 (2003-04-01), pages 416-423, XP002446389, ISSN: 1078-8956, DOI: 10.1038/NM843

## Description

### BACKGROUND OF INVENTION

### Field of the Invention

The present disclosure generally relates to biomarkers and its use to detect or diagnose liver cancer. The scope of protection is defined by the appended claims.

### Description of the Related Art

Hepatocellular carcinoma (HCC) is a type of cancer which is most prevalent in adult liver cancer and is the 3^{rd} leading cause of cancer related death worldwide (Stefaniuk P, et al., 2010, World J Gastroenterol 16: 418-424). Patients with HCC are usually asymptomatic until later stages. Thus the patients frequently miss the optimal timing for a proper treatment and thus usually have a very poor prognosis. Particularly the patients usually die within one year after diagnosis when it is not possible to remove the cancer by surgery. Therefore the patients can be greatly benefited by early diagnosis or detection.

At present, diagnosis of HCC is commonly performed using the serum marker alfa-fetoprotein (AFP) often in combination with ultrasonography. However the concentration of AFP is also found to be increased in non-HCC such as chronic or acute hepatitis and benign tumors. The specificity and sensitivity of AFP to detect HCC is considered to be low and AFP has been found to have about 66% overall sensitivity and 82% overall specificity. Thus AFP is not suitable for all types of liver cancer. AFP concentration of 200 (or 400) ng/mL is used as criteria in combination with other clinical findings in ultrasonography test for diagnosis of HCC. Costantinis et al. 2013 disclose biomarkers for HCC, derived from comparison of the model cell line for HCC, HepG2, and normal hepatocytes. Currently there are no biomarkers developed to accurately detect liver cancer in a person without symptoms and measuring the level of AFP is a main non-invasive method for early detection of liver cancer in people at high risk of developing the same. When AFP was first developed as a marker, the criteria considered having enough specificity and sensitivity to diagnose HCC was AFP level of 20 ng/mL. However the specificity remained at just 60%. The current level suggested by the American Association for the Study of Liver Diseases is 200ng/mL, in which case the specificity is increased. However the sensitivity is very low at just 22%. According to the previous studies, the overall specificity and sensitivity of AFP in diagnosing liver cancer is about 66% and 82%, respectively. Thus AFP has a limitation in applying for all types of liver cancer.

US Patent Application Publication No. 2010/030437 relates to liver cancer diagnosing methods and compositions and discloses methods of diagnosing liver cancer by detecting CpG islands present in BASF1, SPINT2, APC, CCND2, CFTR, RASSF1 and SRD5A2 genes.

KR Patent No.1161789 relates to novel biomarkers for diagnosing liver cancer and its use, and discloses the use of NK4 proteins for the diagnosis or prognosis of liver cancer.

Also other serum markers whose criteria for diagnosing are yet to be determined include such as Descarboxyprothrombin(DCP), Prothrombin Induced by Vitamin K Absence II (PIVKA-II), glycosylated AFP versus total AFP (L3 fraction), alpha fuco-sidase, glypican 3, HSP-70 and the like. However those are used as prognosis markers and not available as a marker for diagnosis due to their low specificity.

Therefore there are urgent needs to develop biomarkers with improved specificity and sensitivity to overcome the limitations of current testing.

### SUMMARY OF THE INVENTION

The present disclosure is to provide biomarkers and is use, in alone or in combinations of markers, in diagnoses hepatocellular carcinoma non-invasively with improved specificity and sensitivity.

In one aspect, the present invention provides one or more biomarkers for diagnosing or prognosis of hepatocellular liver cancer that are selected from the group consisting of ACADVL, ANLN, BASP1, MTHFD1, CAPN1, C4A, FLNB and PABPC1.

The biomarkers according to the present disclosure make possible the accurate and sensitive detection of hepatocellular live cancer, considering the high sensitivity and the specificity and the convenience of the detection provided by the present markers, in alone or in combination with the other established markers and/or testing, for example AFP. In one embodiment, the biomarkers of the present disclosure are used as combination of markers and include ANLN and FLNB; BASP1 and PABPC1; BASP1, PABPC1, and CAPN1; BASP1, PABPC1, CAPN1 and ANLN; BASP1, PABPC1, CAPN1, ANLN and C4A; BASP, PABPC1, CAPN1, ANLN, C4A and MTHFD1; BASP1, PABPC1, CAPN1, ANLN, C4A, MTHFD1 and ACADVL; or BASP1, PABPC1, CAPN1, ANLN, C4A, MTHFD1, ACADVL and FLNB, but are not limited thereto.

The biomarkers according to the present disclosure can be used for diagnosing or prognosis of hepatocellular live cancer by detecting the level (concentration) and/or the presence or absence of the biomarkers at nucleic acid or protein level.

Hence the biomarkers according to the present disclosure can be provided as a kit or a composition for diagnosis or prognosis of hepatocellular liver cancer comprising an agent to detect the present biomarkers.

The kit or composition for HCC detection can analyze the biomarkers in a sample using various detecting methods. In one embodiment, the kits may be provided as for example and ELISA kit, a dip stick rapid kit, a MRM kit, a microarray, a gene amplification or an immunoassay kit.

Various agents may be employed for detecting the present markers and include for example the agents to detect the level (concentration) and/or presence or absence of the biomarkers at nucleic acid protein level. In one embodiment, nucleic acid sequences of the biomarkers according to the present disclosure (genes encoding the present biomarkers), its complimentary sequences, and fragments thereof, or antibodies specific to the biomarker protein that is encoded by the corresponding nucleic acid sequence, antibody fragments thereof, aptamers, avidity multimers and peptidomimetics are included. In other embodiment, the agents for a western blot, an ELISA, a radioimmunoassay, an immunodiffusion assay, an immunoelectrophoresis, an immunostaining, an immunoprecipitation, a complementary fixation test, a FACS, a mass spectrometry and a protein microarray are included. In still other embodiment, a monoclonal antibody, a polyclonal antibody, a substrate, an aptamer, an avimer, a peptidomimetic, a receptor, a ligand and a cofactor are included. In still other embodiment, the agents for a polymerase chain reaction, a reverse transcription polymerase chain reaction, a competitive polymerase chain reaction, a Nuclease protection assay (RNase, S1 nuclease assay), an in situ hybridization assay, a DNA microarray and a northern blot assay are included. In still other embodiment, a primer set, or a probe, or a primer set and a probe that specifically recognizes each biomarker according to the present disclosure are included.

In other aspect, the present disclosure provides methods for detecting the present biomarkers in vitro to diagnose or prognosis HCC. The methods comprise detecting the level or the presence of at least one biomarker selected from the group consisting of ACADVL, ANLN, BASP1, MTHFD1, CAPN1, C4A, FLNB and PABPC1 at a nucleic acid and/or protein level in a sample from a subject in need thereof; and comparing the detection result with that of a corresponding marker from a control sample wherein the change in the concentration and/or the presence of the biomarkers at the nucleic acid and/or protein level in the subject from the control sample indicates the presence of HCC in the subject.

Among the biomarkers according to the present disclosure, the expressions of ACADVL, ANLN, MTHFD1, and FLNB are increased, and the expression of BASP1, CAPN1, C4A, and PABPC1 are decreased when the subject of interest is afflicted with HCC. The present detection method may be performed by employing various analysis methods such as a microarray, a gene amplification, an antigen-antibody reaction or a mass spectrometry. The combinations of makers used for the present method and other analysis methods to be employed are as described hereinbefore and after.

In still other aspect, the present disclosure provides methods for screening an agent for preventing or treating liver cancer, which comprise providing a cell comprising at least one nucleic acid sequence encoding the biomarkers selected from the group consisting of ACADVL, ANLN, BASP1, MTHFD1, CAPN1, C4A, FLNB and PABPC1; contacting the cell with a testing agent; determining the expression level of the gene at a protein and/or mRNA level in the cell treated with the testing agent; comparing the expression level of the cell to that of a control and selecting the testing agent as a potential therapeutic agent when the expression level of the nucleic acid sequence encoding at least one of the markers is decreased in the cell treated with the testing agents, compared to that of the control.

### ADVANTAGEOUS EFFECTS

The biomarkers according to the present disclosure enable, with high accuracy and sensitivity, an early diagnosis or monitoring of hepatocellular carcinoma as well as an effective prognosis or assessment of the progression of the disease. The present methods employing the present markers provide a noninvasive test by using the sample such as blood and the like. The present biomarkers may be developed as a part of POCT device for a convenient and efficient early detection of HCC at home or in the clinics.

### BRIEF DESCRIPTION OF THE DRAWINGS

These and/or other aspects and advantages of the invention will become apparent and more readily appreciated from the following description of the embodiments, taken in conjunction with the accompanying drawings of which:
Fig. 1 shows a schematic description of HCC biomarker discovery process employed in the present disclosure.

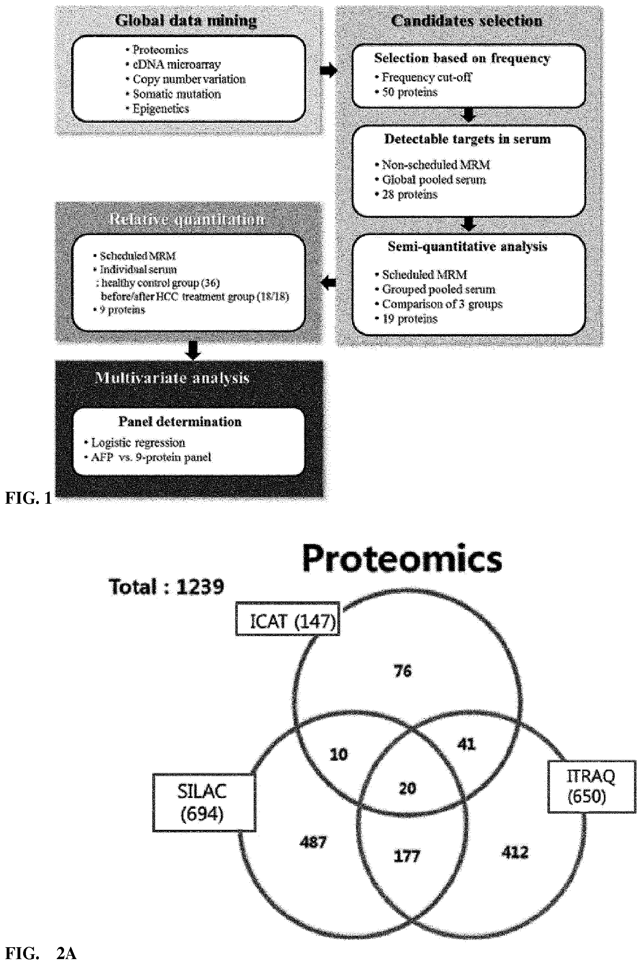

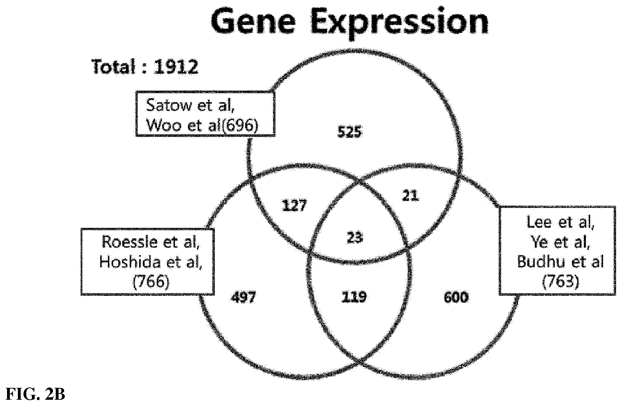

Figs. 2A to 2F show the result of data mining for liver cancer markers. Each figure shows the results of proteome analysis, gene expression analysis, gene copy-number variation, epigenetic analysis, and somatic mutation analysis results and the summary of the above analysis. References in Fig 2B to 2D are as follows: Lee et al., Nature Medicine 2006; 12:410-416; Roessler et al., Cancer Research 2010; 70:10202-10212; Satow et al., Clinical Cancer Research 2010; 16:2518-2528; Chen et al., Hepatology 2010; 52:1690-1701; Chochi et al., J. of Pathology 2009; 217:677-684; Hashimoto et al., Modern Pathology 2004; 17:617-622; Acevedo et al., Cancer Research 2008; 68:2641-2651; Hernandez-Vargas et al., PLos ONE 2010; 5(3): e9749. doi:10.1371/journal.pone.0009749; ZHU, Cell Research 2005; 15:272-280.
Fig. 3 shows a response curve of IS peptide using ATP-dependent RNA helicase A(DHX9) peptide.
Fig. 4A to 4D show the result verified by Western blot of four markers, AFP, ANLN, C4A and FLNB respectively.
Fig 5A to 5C show ROC curves of AFP and 2-marker panel. LR analysis was performed by comparing AFP with the 2-marker panel. Using LR analysis models, false positive/negative rates were prepared in the classifier tables, in which error rates are also shown. LR analyses were performed for 3 types as indicated in each figures.
Fig 6A to 6J show ROC curves of the multi-marker panel combined using LR analysis models. The markers comprised in each combination are indicated in each figure.

### DETAILED DESCRIPTION OF THE EMBODIMENTS

It takes tremendous amount time and efforts to discover protein marker candidate specific to a disease. Thus, as an initial step, the present inventors have performed global data mining with regard to liver cancer in several disciplines including proteomics to select the most significant candidates.

The present invention is based on the findings of 50 most significant proteins in liver cancer which were selected through the data mining of 5 different types of datasets in proteomics, gene expression using cDNA microarray, copy number variation, somatic mutation, and epigenetics, and the findings of the proteins whose expression level have been significantly different between the healthy control group and liver cancer patient groups (untreated and treated).

Therefore in one aspect, the present disclosure relates to at least one biomarker for diagnosing or prognosis of hepatocellular carcinoma selected from the group consisting ACADVL, ANLN, BASP1, MTHFD1, CAPN1, C4A, FLNB and PABPC1.

The term diagnosis as used herein refers to determining the disease or disorder susceptibility of a subject, determining whether a subject has a specific disease or disorder, determining the prognosis (for example, identification of transitional cancer status, stages or progression of a cancer or determining the response to cancer treatments) of a subject who has specific disease or disorder, or therametrics(for example, monitoring the status of a subject to provide the information on the efficacy of treatment).

The term biomarker for diagnosing or diagnosis marker as used herein refers to an agent that may discriminate a liver cancer tissues or cells from normal cells or a treated liver cancer tissues or cells, and comprises an organic and biological molecule and the like, such as proteins or nucleic acid molecules, lipid, glycolipid, and glycoprotein that has increased or decreased in tissues or cells compared with normal control samples. In the present disclosure, the agent is one or more markers selected from the group consisting of long-chain specific acyl-CoA dehydrogenase (ACADVL), actin-binding protein, anillin (ANLN), brain acid-soluble protein 1 (BASP1), c-1-tetrahydrofolate synthase, cytoplasmic (MTHFD1), calpain-1 catalytic subunit (CAPN1), complementary C4-A (C4A), filamin-B (FLNB), and polyadenylate-binding protein 1 (PABPC1) or its corresponding genes or fragments, the expression level of which is increased or decreased at hepatocellular carcinoma tissues or cells. Particularly the expression levels of ACADVL, ANLN, MTHFD1, and FLNB is increased, and that of BASP1, CAPN1, C4A, and PABPC1 is decreased.

In the present disclosure, the present markers may be used in alone or two or more markers may be used in combination to further improve the specificity and/or sensitivity. For example, two, three, four, five, six, seven or more markers may be combined. Further the present markers may also be used together with the conventional markers, such as AFP, and/or with the other diagnosis test available, such as liver ultrasonography. The person skilled in the art would be able to select the combination of markers that show a desired sensitivity and specificity using the methods such as Logistic regression analysis and/or analysis of the biological samples from the subjects including a normal person and patient using the methods such as described in the examples of the present disclosure. In one embodiment of the present disclosure, the combination includes, but is not limited to, for example, ANLN and FLNB; BASP1 and PABPC1; BASP1, PABPC1, and CAPN1; BASP1, PABPC1, CAPN1 and ANLN; BASP1, PABPC1, CAPN1, ANLN and C4A; BASP, PABPC1, CAPN1, ANLN, C4A and MTHFD1; BASP1, PABPC1, CAPN1, ANLN, C4A, MTHFD1 and ACADVL; and BASP1, PABPC1, CAPN1, ANLN, C4A, MTHFD1, ACADVL and FLNB.

The hepatocellular carcinoma (HCC) of the present disclosure is a primary malignant tumor of liver tissue developed in a subject who has risk factors such as alcoholic abuse, viral hepatitis and metabolic liver disease. HCC which usually does not develop fibrous stroma leads to a bleeding and necrosis, and thus resulting in a vascular invasion into hepatic portal vein system which may lead to a hepatorrhexis and abdominal plasma exudation in severe cases.

The biological sample of the present disclosure is a substance or a mixture of the substances that contain or is expected to contain/express one or more of the present biomarkers, and includes cells, tissues or bodily fluids from an organism, particularly human, for example, whole blood, urine, plasma, and serum, but is not limited thereto. Also the sample includes cells or tissues cultured in vitro as well as those derived directly from an organism. Various samples may be used for the detection of HCC markers according to the present disclosure. In one embodiment, urine, whole blood, plasma and/or blood serum can be used. In other embodiment, the liver tissues/cells or in vitro cell cultures from an organism of interest, where HCC has developed or HCC is plausible or likely to be developed, may be used, but the samples are not limited thereto. Also the fractions or derivatives of the blood, cells or tissues are included. When cells or tissues are used, lysates thereof may also be used.

The term detection or detecting as used herein refers to a determination in quantity, quality and/or changes in expression patterns and/or profiles of the expression. The detection includes a determination of the presence and/or absence as well as the levels of the present markers. The present markers may be detected using the methods known in the art, and the person skilled in the art would be easily able to select appropriate methods for the detection.

The markers according to the present disclosure can be detected to analyze the presence or absence of the markers and/or the expression level, the difference in the expression level or the changes in the expression level at mRNA and/or protein level through various quantitative and/or qualitative analysis methods known in the art.

The detection of the markers to diagnose HCC according to the present disclosure may be based on the functional and/or antigenic characteristics thereof. The markers can be detected using the agents that specifically interact with the markers at the nucleic acid level, particularly mRNA level and/or protein level, or the agents that activates the markers may also be used.

In this perspectives, the present disclosure relates to nucleic acid sequences or the sequences complementary thereto or the fragments thereof, or antibodies or aptamers that specifically recognize the protein encoded by the nucleic acid sequences.

The quantitative and qualitative analysis of the makers for HCC diagnosis according to the present disclosure may be done by various methods known in the art that can detect the nucleic acid molecules or proteins quantitatively or qualitatively. For example, the methods include a western blot, an ELISA, a radioimmunoassay, an immunodiffusion, an immunoelectrophoresis, an immunostaining, an immunoprecipitation, a complement fixation assay, an array system such as a nucleic acid array or a protein array such as antigen array, a nucleic acid transcription and amplification system, eTag system, a system based on tailed beads, a binding with a tailed antibody in solution/suspension and a detection by flow cytometry, or a mass spectrometry, and the like. These methods are known and documents such as chip-based capillary electrophoresis: Colyer et al. 1997. J Chromatogr A. 781(1-2):271-6; mass spectroscopy: Petricoin et al. 2002. Lancet 359: 572-77; eTag systems: Chan-Hui et al. 2004. Clinical Immunology 111:162-174; microparticle-enhanced nephelometric immunoassay: Montagne et al. 1992. Eur J Clin Chem Clin Biochem. 30:217-22 may be referred.

In one embodiment of the present disclosure, a mass spectrometry is used for detecting the present markers, wherein the proteins are extracted from the appropriate samples and analyzed using the method such as described in the Examples of the present disclosure, and in the literatures Kim, et al. 2010 J Proteome Res. 9: 689-99; Anderson, L et al. 2006. Mol Cell Proteomics 5: 573-88 may also be referred. In one embodiment Multiple Reaction Monitoring (MRM) technology utilizing Triple Quadrupole LC-MS/MS and QTRAP and the like may be used. MRM is a method for exactly quantifying multiple markers present in biological samples in minute amount. In MRM, by a first mass filter (Q1), parent or precursor ions are selected from the ion fragments generated in ionization source and transferred to a collision cell. And then the precursor ions arrived at the collision cell collide with internal collision gas, and are fragmented into products or daughter ions and transferred to a second mass filter (Q2), from which only the specific ions are delivered to a detector. In this way only the information of the desired target can be obtained with high selectivity and sensitivity. The literature Gillette et al., 2013, Nature Methods 10:28-34 and the like may be referred.

In other embodiment, the agents that specifically recognize or bind to the present protein markers or mRNA or DNA encoding the same or the arrays comprising such agents may be used.

In a still other embodiment, an immunoassay using sandwich system like ELISA (Enzyme Linked Immuno Sorbent Assay), or RIA (Radio Immuno Assay) and the like may be used for quantitative and/or qualitative detection of the present markers. In this system, the biological samples are reacted with a first antibody fixed to a solid substrate/support such as a glass, a plastic (for example, polystyrene), polysaccharides, a bead, a nylon or nitrocellulose membrane or a microplate well to form a complex and the complex is then allowed to react with an second antibody that is usually labeled with agents that can be detected directly or indirectly such as radioactive substances like ³H or ¹²⁵I, fluorescent materials, chemiluminescent substances, hapten, biotin, or digoxygenin and the like. In some cases, the labeling materials are conjugated with an enzyme such as horseradish peroxidase, alkaline phosphatase, or maleate dehydrogenase that is able to produce colors or color changes or illuminate in the presence of appropriate substrates.

Other methods based on immune reaction may also be used. In other embodiment, an Immuno Electrophoresis such as an Ouchterlony plate, a Western blot, a Crossed IE, a Rocket IE, a Fused Rocket IE, or an Affinity IE, which can detect the markers simply by antigen-antibody reaction may be used. The agents or materials that may be used in the methods described above are known the art. For example, the markers may be detected through an antigen-antibody reaction, or a reaction with a substrate, nucleic acid or peptide aptamers, receptors or ligands that specifically recognize the present markers, or cofactors or using mass spectrometry. The agents or materials that bind or interact specifically with the markers of the present disclosure can be utilized by means of chip or with nanoparticles. The immunoassay or immunostaining methods as described above are disclosed in the following literatures : Enzyme Immunoassay, E. T. Maggio, ed., CRC Press, Boca Raton, Florida, 1980; Gaastra, W., Enzyme-linked immunosorbent assay(ELISA), in Methods in Molecular Biology, Vol. 1, Walker, J.M. ed., Humana Press, NJ, 1984 etc. The intensities of the signals generated by the immunoassay mentioned above are then analyzed, namely compared with the signals from appropriate controls for the determination whether the sample has HCC or not.

Each marker of the present disclosure can be used for diagnosis or prognosis of HCC by detecting the marker(s) quantitatively and/or qualitatively at nucleic acid level, particularly at mRNA level. Various methods known in the relevant art may be used for this purpose. For example, for the quantitative or qualitative detection of the marker(s) or the expression pattern thereof at mRNA level, a polymerase chain reaction (PCR) combined with cDNA synthesis, a RT-PCR (reverse Transcription PCR), a competitive RT-PCR, a real-time RT-PCR, a Nuclease Protection Assay (NPA) such as a RNase or a S1 nuclease assay, an in situ hybridization assay, a DNA microarray or chip or Northern blot and the like may be used, the methods of which are known in the art and also can be performed using commercially available kits. The skilled person in the art would be able to select appropriate methods for practicing the present invention. For example, the transcriptome size that exists in cells can be determined by a northern blot, which has an advantage of being able to use diverse probes. A NPA is useful for multi-marker analysis. An in situ hybridization is useful for localizing the transcriptome such as mRNA in cells or tissues. A RT-PCR is particularly useful when small amount of samples are employed.

The reagents for detecting the presence markers in the amount or for determining the pattern of mRNA via RT-PCR comprise, for example, a primer set and/or probe specific to the marker(s) of the present disclosure. The terms "primer" or "probe" refers to a nucleic acid sequences that can bind to a template via complementarity and have free 3'-hydroxy group that can serves as a starting point for polymerization using the template by reverse transcriptase or DNA polymerases. The reagents used herein can also be labeled with appropriate materials for the signal detection such as color forming agent, illuminating or fluorescent materials. In one embodiment, a northern blot or a reverse-transcription PCR is used for the mRNA detection. The latter is a method to detect or determine the expression level of a specific gene expression in a sample, wherein the RNA, particularly mRNAs are isolated from the sample and cDNAs are synthesized from the isolated RNA, and then the cDNAs are amplified using a specific set of primers, or a combination of primers and probe. Such methods to detect the presence/absence or quantity of a specific gene or marker may be found in for example, Han, H. et al, 2002. Cancer Res. 62: 2890-6.

In other aspect, the present disclosure provides a kit, composition or system for diagnosing or prognosis of hepatocellular carcinoma comprising an agent to detect the expression level or presence of at least one biomarker selected from the group consisting of ACADVL, ANLN, BASP1, MTHFD1, CAPN1, C4A, FLNB and PABPC1. The detection agents and the methods of using the agent are described hereinbefore. The agents for detecting the present marker(s) can be provided in a container separated in sections, or can be provided separately. In this regards, the present disclosure provides a device that provides the agents for detecting the present markers in separate sections in a container.

In one example, the detecting agents are antibodies specifically binding to one or more markers of the present disclosure, and can detect the protein in a sample such as serum quantitatively and/or qualitatively. In another example, the antibodies may be provided as conjugated to a substrate or support, for example to a well of the multi-well plates or a surface of the glass slides or nitrocellulose filters.

In other aspect, the detecting agents which may be used for the present disclosure may be provided as a type of array such as microarray or chip, and the references for the array preparation technology may be found in for example, Schena et al., 1996, Proc Natl Acad Sci USA. 93(20):10614-9; Schena et al., 1995, Science 270(5235):467-70; and U.S. Pat. Nos. 5,599,695, 5,556,752 and 5,631,734. The detecting agents that can be provided as attached to an array include, but are not limited to, antibodies specifically recognizing the present marker, or antibody fragments, or aptamers, avimers(avidity multimer) or peptidomimetics.

The detecting agents may also be labeled for the direct or indirect signal detection via sandwich forms. In the case of direct labeling method, biological samples such as serum to be used for array may be labeled with a fluorescent material such as Cy3 and Cy5. In the case of indirect labeling, unlabeled samples are allowed to bind to the detecting agent which is attached to the array, and then the target or marker proteins are detected by the labeled antibodies which specifically recognize the marker or target. In the case of sandwich methods, the sensitivity and specificity of the detection is usually high and able to detect the marker at the pg/mL level. In addition, labeling agents including such as radioactive materials, agents that produce visible colors under proper condition, magnetic particles and high-density electron particle and the like may also be used.

For the detection of fluorescence signals, a scanning confocal microscopy may be used, which are available from for example, Affymetrix, Inc. or Agilent Technologies, Inc. and the like.

The kits or compositions of the present disclosure may also include one or more additional components as needed for the detection of the present marker(s), which include, for example, binding buffers, reagents for preparing biological samples, syringes for blood collecting or negative and/or positive controls.

The kits or compositions of the present disclosure in which various agents as described above may be contained may be provided for an ELISA, a dip stick rapid kit, a MRM, microarray, a gene amplification, or an immunoassay. The reagents which may be included for a particular kit may be appropriately selected from the known agents in view the present disclosure.

In one embodiment, an ELISA or a dip stick rapid kit is used, and in this case, the antibodies to detect the present markers may be provided as conjugated to a substrate/support, for example to wells of a multi-well plate or surface of a glass slide or a nitrocellulose paper. A dip stick is a form widely used in POCT (point of care technology) in which the biomarkers of the present disclosure may be detected by employing one or more antibodies that are conjugated to a substrate such as nitrocellulose paper which is then contacted with a sample such as serum for example by dipping one end of the stick to the serum and then the sample is moved through the substrate by a capillary action, and the markers are detected by a color that is developed when the markers of interest are bound by the antibody attached to the substrate.

In other embodiment, a MRM kit which is based on peptide analysis is provided. MRM analysis may be performed or used as described above. The MRM is a method using the peptides that selectively recognize a particular protein, and can detect the markers in biological samples in a more stable manner compared to the existing methods that employ antibodies that are sensitive to environmental conditions such as temperature and humidity and the like.

Also, the kits of the present disclosure may also include instructions for using the kit to detect the markers according to the present disclosure.

In still other aspect, the present disclosure provides compositions comprising at least one biomarker selected from the group consisting ACADVL, ANLN, BASP1, MTHFD1, CAPN1, C4A, FLNB and PABPC1, and/or the agents that are able to detect the markers.

In still other aspect, the present disclosure provides methods for diagnosing or prognosis of HCC, or detecting in vitro at least one maker of the present disclosure for diagnosis or prognosis of HCC, which comprises detecting the presence or amount of at least one biomarker selected from the group consisting of ACADVL, ANLN, BASP1, MTHFD1, CAPN1, C4A, FLNB and PABPC1 at a nucleic acid and/or protein level in a sample from a subject in need thereof.

In one embodiment, the present methods may additionally include a step of comparing the detection result of the subject sample to that of a corresponding marker in a control sample from healthy subject wherein the change in the amount and/or in the presence or absence of the biomarker at the nucleic acid and/or protein level in the subject from the control sample indicates that the subject has HCC. In one embodiment, the present biomarkers ACADVL, ANLN, MTHFD1 and FLNB are found to be increased in HCC sample and the biomarkers BASP1, CAPN1, C4A and PABPC1 are found to be decreased in HCC sample. Thus the subject can be diagnosed accordingly based on the detection result.

Samples such as biological samples, control or reference groups employed in the present methods, methods for determining the present biomarkers and the reagent used therefor, and methods for data analysis required for the interpretation or diagnosis are as described hereinbefore and in the following Examples.

The subject to be diagnosed by the present methods is a mammal, particularly human. The subjects include ones who are suspected of HCC or at high risk or others who need for HCC diagnosis or prognosis. In one embodiment, the present methods are applied for a subject who has symptoms indicative of liver related disease, such as an abdominal pain, hepatomegaly, ascites, jaundice, muscle wasting, hepatitis(for example HCV infection), or esophageal varix and the like. In other embodiment, the subject includes ones who appear to be normal and yet to develop HCC related symptoms.

In other embodiment, the subject may be a person whose concentration of serum AFP is low or in a normal range and thus not diagnosed as HCC by applying AFP criteria. In this case, the serum concentration of AFP may be about 0 µg/l (not detected) to 20 µg/l, e.g., 0 µg/l (not detected) to 5 µg/l, 5 µg/l to 10 µg/l, 10 µg/l to 15 µg/l, or 15 µg/l to 20 µg/l.

When the combinations of two or more markers are used for the present methods, a profile, namely a set of data including quantitative information in relation to the protein expressions in the sample of interest may be generated.

When the profiles are obtained using the present markers, HCC in the sample of a subject may be diagnosed or determined by comparing the result of the sample with that of the control or reference groups. The control or reference groups which may be used for the present methods include a negative control from a healthy or normal subject, or from a subject cured of HCC, and a positive control is from HCC patient diagnosed not by the present method but by the other methods, or from liver cirrhosis patient, or from hepatitis patient.

In one embodiment, as a control or reference groups, normal samples or samples from HCC-treated patient are used and compared with the acquired profile from the subject who needs diagnosis.

For comparing the profiles obtained, methods known in the art may be used. For example, comparison of the digital images of expression profiles, comparison using expression database, or US Patent NOs 6,308,170 and 6,228,575 may be referred.

The acquired profiles using the present markers may be processed by data analysis methods known in the art. For example, the methods include but are not limited to nearest neighbor classifier, partial-least squares, SVM, AdaBoost and clustering-based classification. Or the methods described in the literatures such as Ben-Dor et al (2007, J. Comput. Biol. 7: 559-83), Nguyen et al (2002, Bioinformatics 18:39-50), Wang et al (2003, BMC Bioinformatics 4:60), Liu et al (2001, Genome Inform. Ser. Workshop Genome Inform. 12:14-23), Yeang et al (2001, Bioinformatics 17 Suppl 1:S316-22) and Xiong (2000, Biotechniques 29(6):1264-8, 1270) and the like may also be referred.

Further, various statistical analysis methods may be employed to calculate the significance of the data obtained using the present markers to diagnose or prognosis HCC. In one embodiment, a logic regression method is used for a statistical analysis and the literatures such as Ruczinski, 2003, Journal of Computational and Graphical Statistics 12:475-512 may be referred. A logic regression method is similar to CART method in which classifier is presented as a binary tree, but Boolean operators are used for each node that is more general and related to characteristics than "and" operator used in CART. There are other analysis methods such as nearest shrunken centroids (Tibshirani. 2002 PNAS. 99:6567-72), random forests (Breiman. 2001. Machine Learning 45:5-32 and MART (Hastie. 2001. The Elements of Statistical Learning, Springer).

In one embodiment, the confidence level of a significant difference between the test sample and the controls to diagnose the sample as HCC may be determined in a statistical analysis. The raw data used for a statistics are the values analyzed in duplicate, triplicate or more for each marker. It can be determined that the sample of interest is to be significantly different from the normal control when the values of at least one or at least two markers from ACADVL, ANLN, BASP1, MTHFD1, CAPN1, C4A, FLNB and PABPC1 in the sample of interest are found to be higher than the preset value to be significant. In another example, the sample of interest is to be significantly different from the normal control when the markers ANLN and/or FLNB are significantly higher than the preset value.

This statistical analysis is useful to decide the clinical significance of the clinical and genetic data as well as the data produced from the biomarkers.

Also the present methods may be used to determine the severity of the HCC. For example, it may be diagnosed as a mild HCC, a moderate HCC or a severe HCC by comparing the data obtained with the profiles form the positive and/or negative control groups. Further, the marker profiles obtained from a certain HCC groups may be classified according to a certain criteria. In this way, it is possible to find the disease at the early stage so that expensive test such as magnetic resonance imaging (MRI) may not be needed.

In other embodiment, the present method may be performed multiple times with a certain interval over a period of time for example over one year to monitor the changes in the expression pattern of the present markers. The increase or decrease in the expression depending on the markers can be used to relate the sample with a particular status or severity of HCC. Also in one subject, the data may be used to determine the development, progression, and/or aggravation of HCC over time in comparison to the previous data from the same subject or to the data from the controls. The changes in the expression pattern or levels over time thus can be used to treat HCC or to prevent the progression of HCC into a more advanced stage. Also the present methods may be used together with conventional diagnosis methods such as AFP test, a ultrasonography, a computerized axial tomography(CT scan) or a magnetic resonance imaging (MRI).

In other aspect, the present disclosure relates to methods of screening an agent for treating or preventing HCC or symptoms associated therewith. The agents that are able to change or affect or influence the expression level or pattern of the present markers in HCC tissues or cells can be selected as candidates for HCC therapeutic agent. In one embodiment, the present screening method comprises providing a cell including at least one gene selected from the group consisting of ACADVL, ANLN, BASP1, MTHFD1, CAPN1, C4A, FLNB; contacting the cell with a testing agent; determining the expression level of the gene at the protein and/or mRNA level in the cell treated with the testing agent; comparing the expression level of the cell to that of a control and selecting the testing agent as a potential therapeutic agent when the cells treated with the testing agent shows a decreased level of expression of the markers compared to that of the controls.

In one example, small molecules are used to identify agents useful for treating and/or preventing HCC or symptoms associated therewith. For example small molecules with molecular weight of about less than 1,000Da such as 400 Da, 600 Da, or 800Da are used. If desired, small molecules may form part of a library, the total number of small molecules included therein may vary from dozens to millions. Test substance of a library may include peptides, peptoides, circular or liner oligomeric compounds, template based compounds such as benzodiazepine, hydantoin, biaryls, carbocyclic and polycyclic compounds such as naphthalene, phenothiazine, acridine, steroids and the like, carbohydrate and amino acid derivatives, dihydropyridine, benzhydryl and heterocyclic compounds such as triazine, indole, thiazolidine and the like, but are not limited thereto.

The present disclosure is further explained in more detail with reference to the following examples. These examples, however, should not be interpreted as limiting the scope of the present invention in any manner.

### EXAMPLES

In the present examples, the expression levels of the 50 proteins originated from the serum samples of 3 subject groups (healthy control group, untreated HCC group and treated HCC group) were verified via multiple reaction monitoring (MRM) using mass spectrometry(Triple Quadrupole LC-MS/MS), and the markers for HCC that have shown significant differences between the groups were discovered.

### Example 1. Materials and Methods

### 1-1. Clinical sample information

The institutional review board of Seoul National University Hospital (approval No. H-1103-056-355) approved the protocol of the present invention, and the written informed consent was obtained from each patient or their legally authorized representative. The clinical characteristics of the patients are as in Table 1

In the present invention, 36 samples from a healthy control group (male/female = 18/18), 18 untreated HCC samples from a patient group diagnosed with HCC (male/female = 13/5) and 18 treated HCC samples from HCC patients who underwent HCC therapy (male/female = 13/5) were used. Untreated and treated HCC samples were collected from the same patients. The HCC serum samples were collected before the start of treatment for liver cancer, and then the treated samples were collected and used for the analysis only when liver cancer wasn't recurred and metastasized and completely cured based on the data followed for more than a year after the treatment.

The rates of liver cancer is 70% more prevalent in male than in female, so the samples were collected to include 70% more male samples than the female samples. To avoid the complexity of the data analysis arising from the nature of the samples used, the samples from the patients who had been treated only with transarterial chemoembolization (TACE) and percutaneous ethanol injection (PEIT) were used for the analysis. Besides, Virus (HBV, HCV) and alcohol are known as the two main cause of liver cancer, but the recent study showed that in Asia and Africa, 70% of liver cancer is arise from HBV infection and 20% from HCV and 10 % from alcohols. Based on this report, the samples of liver cancer only form HBV infection which is the most prevalent cause in Asia were used for the study. The clinical information of the samples used is as follows.

**[Table 1]**

| **Clinical characteristics of the subject** | | | | |
|---|---|---|---|---|
| | **MRM analysis** | | **Western blot analysis** | |
| | **Untreated HOC group and corresponding treated HCC Group** | **Healthy control group** | **Untreated HCC group and corresponding treated HOC Group** | **Healthy control group** |
| **Total patient number** | 18 in each group | 36 | 13 in each group | 13 |
| **Gender (Male/Female)** | 5 / 13 | 18 / 18 | 10 / 3 | 8/5 |
| **Age (Mean, Range)** | 60.6 (47-81) | 58.7 (50-69) | 62.4 (48-79) | 56.2 (52-67) |
| **Etiology of river disease** | HBV, 18 (100%) | | HBV, 13 (100%) | |
| **Locoregional modality** | | | | |
| TACE | 7 | | 4 | |
| PEIT | 11 | | 9 | |
| **AFP value (Mean,Range)** | 10794 (14.1 - 6900] | | 245.2 (16-730) | |
| <20 ng/ml | 2 | | 1 | |
| 20-200 ng/ml | 4 | | 6 | |
| 200-1000 ng/ml | 7 | | 6 | |
| >1000 ng/ml | 5 | | 0 | |
| **PIVKA value(Mean,Range)*** | 916 (5-10720) | | 117.6 (28-612) | |
| <20 ng/ml | 4 | | 0 | |
| 20-100 ng/ml | 6 | | 7 | |
| 100-1000 ng/ml | 3 | | 6 | |
| >1000 ng/ml | 3 | | 0 | |

| | | | | |
|---|---|---|---|---|
| "PIVKA values were provided for 16 (M11/F5) among a tatal of 18 untreated HCC group Abbreviationsn: AFP : Alpha-Fetoprotein PIVKA : Protein induced by vitamin K absence or antagonist TACE: Transcatheter arterial chemoembolization PEIT :Percutaneous ethanol injection therapy | | | | |

The blood samples were collected and centrifuged immediately at 3000 rpm for 10 min at 4°C to fractionate the serum. The resulting supernatants were then aliquoted(50*µ*ℓ) and stored at -80°C until analysis.

### 1-2. Tryptic Digestion of serum samples

Serum proteins were treated for MRM analysis as follows. Serum proteins were quantified by bicinchoninic acid (BCA) assay. The 200*µ*g aliquots of the serum samples were denatured with 6 M urea, 50 mM Tris, pH 8.0, and 30 mM dithiothreitol (DTT) at 37°C for 60 min and alkylated with 50 mM iodoacetamide (IAA) at room temperature in the dark for 30 min. The urea was diluted 15-fold with 50 mM Tris, pH 8.0 prior to overnight digestion at 37°C with trypsin (Promega, sequencing-grade modified) using a 1:50 (w/w) enzyme-to-serum concentration ratio.

Tryptic digestion was stopped with formic acid at a final concentration of 1% and desalted on Sep-pak tC18 cartridges (Waters Corp., USA). The Sep-pak tC18 cartridges were equilibrated sequentially with 3 ml methanol and 5 ml water that contained 0.1% trifluoroacetic acid (TFA) prior to loading of the tryptic digestion. The cartridges were washed with 3 ml 0.1% trifluoroacetic acid (TFA) and eluted with 1 ml of 60% ACN, 0.1 % TFA. The eluted samples were frozen and lyophilized on a speed vacuum. Prior to MRM analysis, the samples were reconstituted in 0.1% formic acid to 2 µg/µl.

### 1-3. Standard peptide

Peak areas for the MRM runs were normalized using a standard peptide. A synthetic stable isotope-labeled peptide from ATP-dependent RNA helicase A (DHX9) (sequence ELDALDANDELTPLG***R***) with a C-terminal ¹⁵N-, ¹³C-labeled arginine residue (≥ 99 atom % isotopic enrichment) (21^{st} Century Biochemicals Inc., MA) was added to the HCC serum peptide mixture before MRM analysis at 50 fmol. After MRM analysis, the peak area of the internal standard peptide in each sample was used to normalize of the peak area of each target peptide in each MRM run.

### 1-4. MRM

For each target protein, we selected peptides and fragment ions for MRM using Skyline (http://proteome.gs.washington.edu/software/skyline), an open-source software application for developing MRM methods and analyzing MRM data (Stergachis AB, et al., 2011, Nat Methods 8: 1041-1043)

In brief, the full-length protein sequences were imported into Skyline in FASTA format and designed into peptides, each with a list of product ions for monitoring by MRM. In selecting transitions through Skyline, the peptide filter condition was as follows: maximum length of peptide of 20, including at least 8 amino acids. Peptides with repeat arginines (Arg, R) or lysines (Lys, K) were discarded. If methionine (Met, M) was included in the peptide, it was discarded to avoid the risk of modification. If pro-line (Pro) lay next to arginine (Arg, R) or lysine (Lys, K), the peptide was discarded. If a peptide contained histidine (His, H), it was discarded to avoid the risk of charge alteration.

Peptides that satisfied these conditions were used as Q1 transitions. Next, we selected a maximum of 5 Q3 transitions from the fragmentation ions that were derived from the Q1 transitions in descending order. The .csv output files that contained the transition information were exported for MRM into Analyst (AB SCIEX, Foster City, MA), and MRM was run using the nonscheduled MRM method. The MRM data were generated in.wiff and wiff.scan files, which were converted to mzXML format using mzWiff and input into Skyline for MRM data processing, such as selecting the peak intensities of MRM transitions.

### 1-5. Quantification by multiple reaction monitoring

MRM was performed on a nano LC system, which was connected to a hybrid triple quadrupole/ion trap mass spectrometer (4000 QTRAP, AB SCIEX, Foster City, CA) that was equipped with a nanoelectrospray interface. The 4000 QTRAP was operated in positive ion MRM mode, in which Q1 and Q3 were set to transmit different precursor/product ion pairs.

The LC buffer system used was as follows: mobile phase A, 2% acetonitrile/0.1% formic acid and mobile phase B, 98% acetonitrile/0.1% formic acid. The peptides were separated and eluted at a flow rate of 300 ml/min on a linear gradient of mobile phase B from 2% to 40% B in 43 min. The gradient was ramped up to 70% B for 5 min and 2% B for 10 min to equilibrate the column for the next run. The total LC run time was 60 min. The analytical column was 75 µm × 15 cm, packed with Magic C18AQ resin (5 µm, 100 Å, Michrom Bioresources).

Typical instrument settings were as follows: ion spray (IS) voltage of 2.3 kV, an interface heater temperature of 200°C, a GS1 (nebulizer gas) setting of 12, and curtain gas set to 15. MS parameters for declustering potential (DP) and collision energy (CE) were determined by linear regression of previously optimized values in Skyline. MRM experiments were performed with a scan time of 50 ms and scan width of 0.002 m/z, using a unit resolution of 0.7 Da (FWHM) for Q1 and Q3. In the MRM runs, scan time was maintained at 50 ms for each transition, and the pause between transition scans was set to 3 ms. MRM transitions were selected to monitor the fragment ion with the highest intensity. Preference for MRM transitions was given to fragment ions with m/z values that were higher than that of the parent (Kirkpatrick DS, et al., 2005, Methods 35: 265-273).

The optimal transitions for each targeted peptide were determined empirically using the heavy isotope-labeled synthetic reference peptides. To normalize the amount of each protein, 50 fmol of heavy isotope-labeled forms of the peptides in 0.1% formic acid was injected with all serum samples.

### 1-6. Statistical analysis for verification of biomarker candidates

Raw data files from the MRM analysis were processed using Skyline. For each targeted peptide, the results of the peak area integration were inspected manually to correct wrong integrations. The default peak integration and Savitzky-Golay smoothing algorithm were applied. Peptides with at least 3-fold signal-to-noise ratios were considered detectable.

Two approaches were used to assess HCC candidate proteins. First, we distinguished disease group (untreated HCC group) from nondisease group (healthy control group, treated HCC group). Comparisons between the untreated HCC group (n=18) versus healthy control group (n=36) and untreated HCC group (n=18) versus treated HCC group (n=18) were made using analysis of variance (ANOVA). Based on ANOVA, we selected target peptides that had a significance level below 0.05 in mean intensity level between groups.

Second, to evaluate the efficacy of serum biomarkers in distinguishing disease group from nondisease group, we analyzed receiver operator characteristic (ROC) curves and scatter plots. ROC curves and scatter plots were used to determine the optimal cutoff point that yielded the highest total accuracy with respect to discriminating sample groups. The AUC is equal to the probability that a classifier will rank a randomly chosen positive instance higher than a randomly chosen negative one. We performed all statistical analyses and generated all scatter plots and ROC curves with MedCalc (MedCalc, Mariakerke, Belgium, version 12.2.1).

### 1-7. Western blot analysis

To confirm the quantification accuracy of the MRM results, following 9 proteins were analyzed using Western blot: long-chain specific acyl-CoA dehydrogenase, mitochondria (ACADVL), actin-binding protein, anillin (ANLN), brain acid-soluble protein 1 (BASP1), c-1-tetrahydrofolate synthase, cytoplasmic (MTHFD1), calpain-1 catalytic subunit (CAPN1), complementary C4-A (C4A), alpha-fetoprotein (AFP), filamin-B (FLNB), and polyadenylate-binding protein 1 (PABPC1).

The clinical samples in the Western blot experiment is consisted of 13 individual samples from the healthy control group, untreated HCC group, and treated HCC group (Table 1). Serum sample concentrations were determined by BCA protein assay. Equal amounts of protein (30 µg) were mixed with SDS loading buffer (62 mM Tris-HCl, pH 6.8, 10% glycerol, 2% SDS, 2% β-mercaptoethanol, and bromophenol blue), boiled for 10 min, and separated by 15-well-comb SDS-PAGE on a 12% acrylamide gel. After separation, serum samples were transferred to polyvinylidene difluoride (PVDF) membranes (Bio-Rad, Cat. #162-0177), which were blocked with 5% BSA (w/v) in TBS-T (25 mM Tris, pH 7.5, 150 mM NaCl, and 0.05% (w/v) Tween-20) for 2 hr at room temperature.

Membranes were incubated overnight at 4°C with individual primary antibodies (diluted 1:100 to 1:1000). Membranes were washed 5 times with TBS-T and incubated for 2 hr with the appropriate secondary peroxidase-conjugated antibody (1:5000, Santa Cruz Biotechnology, USA). The membranes were then washed 5 times with TBS-T, and target protein bands were visualized using the Chemiluminescent Substrate Kit (Gen-DEPOT, W3651-012). Western blot band intensities were quantified using Multi Gauge. (Fujifilm, ScienceLab 2005, version 3.0). Pooled serum mixture was used as a loading control. All blots were normalized to the band intensity of the pooled serum. Band intensities were analyzed by T-test to identify meaningful differences between sample groups.

### 1-8. Statistical analysis to construct multimarker panel

In this study, we compared non-disease group (healthy control group, treated HCC group) with the disease group (untreated HCC group) using multimarker panel proteins. Logistic regression (LR) analysis was performed to generate the multimarker panel, consisting of several individual markers that differentiated cancerous from non-cancerous subjects. The discriminatory power was examined in 3 situations: healthy control group versus healthy control group, untreated HCC group versus treated HCC group, and healthy control group plus treated HCC group versus untreated HCC group.

LR and ROC curves were constructed using MedCalc, and the analysis was performed as follows: Case 1 was healthy control group versus untreated HCC group, Case 2 was untreated HCC group versus treated HCC group, and Case 3 of healthy control group plus treated HCC group versus untreated HCC group. The "Enter" method was used, and the classification table cutoff value was set to 0.5.

ROC curves were used to evaluate the efficacy of the multimarker panel-in this case, a 2-marker panel. AUC values for individual markers and the 2-marker panel were calculated to examine the distinguishing power of various combinations of HCC candidate markers. Multicolinearity of the panel was checked using IBM SPSS Statistics (version 20, commuter license).

### Example 2. Candidate biomarker selection using global data mining

To obtain a list of biomarker candidate proteins requires tremendous amount of time and efforts. Thus established liver cancer-specific markers were used to avoid redundant experiments to save time and thus to find new markers more rapidly. Thus, the present inventors have performed global data mining with regard to liver cancer in several disciplines as well as proteomics to select the most significant candidates.

With regard to HCC, data mining in proteomics, cDNA microarray, copy number variation, epigenetics, and somatic mutation data were processed. The term "frequency" was used for each target protein. Frequency was defined as the total number of occurrences in 5 biological fields. As a result of data mining in the 5 areas, 5,222 liver cancer-related proteins were selected and filtered by prioritizing candidate proteins. The top 50 high-frequency proteins were chosen and examined with regard to whether they were secreted into plasma, and final candidates were selected by confirmation with the Plasma Proteome Database (http://www.plasmaproteomedatabase.org). Then, sequence files of the 50 selected candidates were prepared in FASTA format and harvested using the Uniprot website (http://*www.uniprot.org*)*.* The 50 FASTA files were input into Skyline to generate theoretical transitions for the MRM analysis. The overall scheme for this study is outlined in Fig. 1.

### 2-1. Data mining of proteomic research

Proteomic studies were screened to determine whether they described quantitative analyses that were based on labeling methods, such as ICAT, SILAC, and iTRAQ, using liver cancer cells, tissues, and human blood, excluding label-free methods. Ten research papers, all published after 2004, were selected with impact factors above 4.0. Analyses that were based on unlabeling methods were excluded. All proteins in the 10 journals put together and analyzed in Ben diagram. The result is shown in Fig.2a.

### 2-2. Data mining of gene expression

cDNA microarray research papers that examined gene expression using liver cancer and control tissues were examined. Nine such reports were selected (all published after 2003), with impact factors above 6.7. using these reports, the data mining of gene expression was processed and the result is shown in Fig.2b.

### 2-3. Data mining of copy number variation research

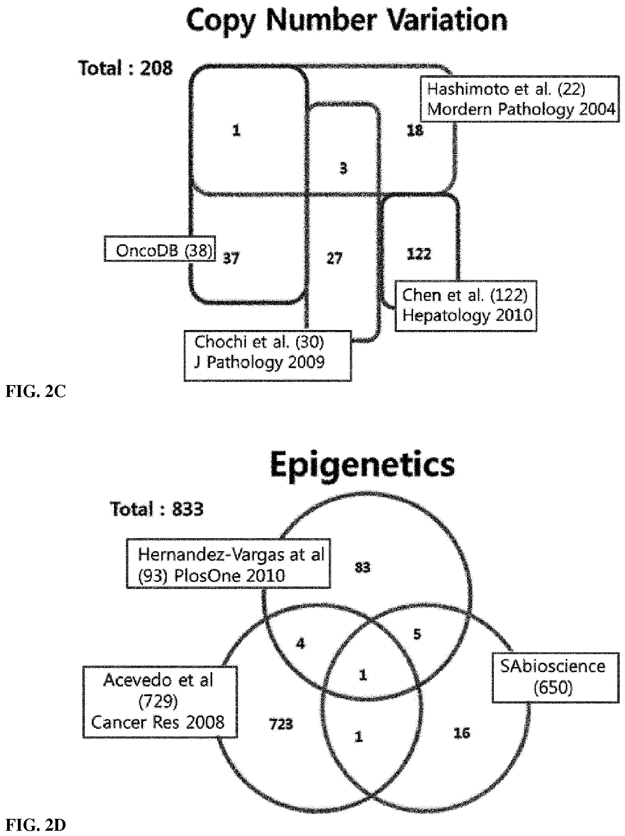

Earlier publications on copy number variation (CNV) by amplification or mutation of liver cancer were investigated, yielding 3 papers after 2004 with impact factors above 4.4. OncoDB (http://oncodb.hcc.ibms.sinica.edu.tw/index.htm) was also used to report copy number variation. Using the 3 papers and OncoDB, the data mining copy number variation research was processed and the result is shown in Fig.2c.

### 2-4. Data mining of epigenetic research

We selected 3 studies from after 2005 with impact factors above 4.3, in which DNA methylation of CpG islands in liver cancer is disclosed. Using the 3 studies, the data mining of epigenetic research was processed and the result is shown in Fig.2d.

### 2-5. Data mining of somatic mutation research

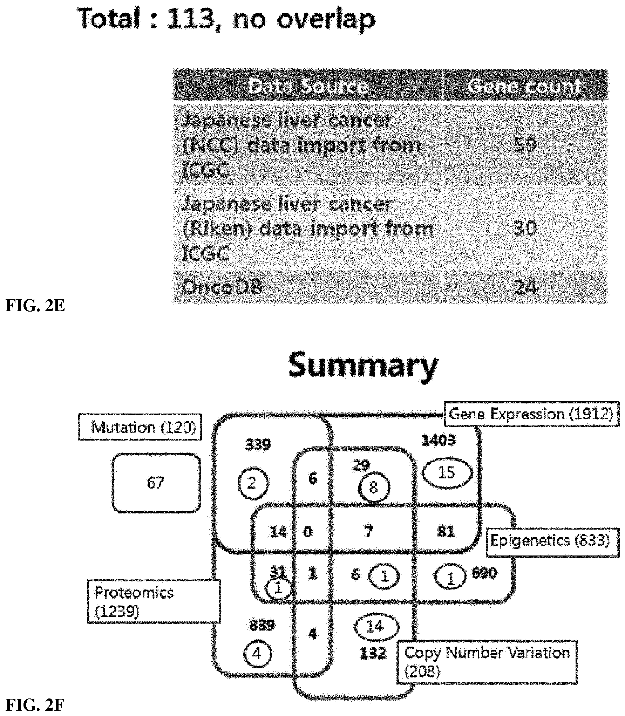

Using the OncoDB (http://oncodb.hcc.ibms.sinica.edu.tw/index.htm), Japan Liver Cancer (NCC, Riken), and International Cancer Genome Consortium (http://www.icgc.org/icgc/cgp/66/420/824) databases, the data mining of somatic mutation research was processed and the result is shown in Fig.2e.

Summary of above data mining results is shown in Fig. 2f.

### Example 3. Examination of the linearity of the standard peptide

The peak area of the internal standard peptide in each sample was used to normalize that of each target peptide in each MRM run. Thus, to assess the quantitative linearity of MRM, a response curve of the standard peptide (sequence of ELDALDANDELTPLG***R***, ATP-dependent RNA helicase A (DHX9)) in the presence of matrix peptides was generated. The standard peptide was diluted serially to 1, 5, 10, 50, 100, 250, and 500 fmol in 200 µg of serum peptide mixture, which is similar to the MRM conditions for target peptides. Further, to verify the endogenous signal of the target peptide, a blank sample that lacked the internal standard peptide was run. Each MRM analysis was performed three times repeatedly.

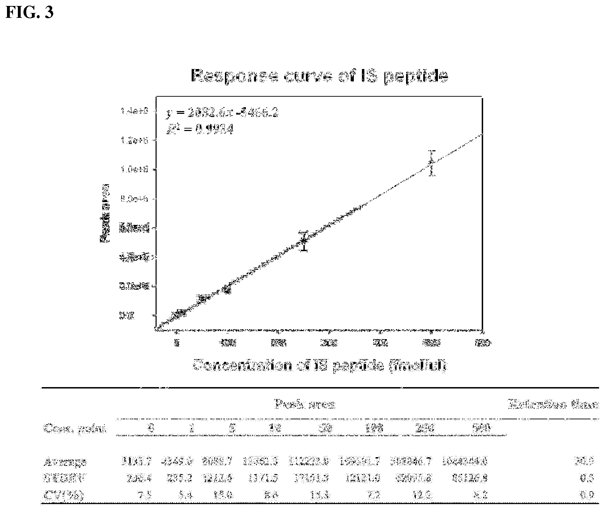

The result is shown in Fig 3. The CV% of the experiment was below 20%, and the correlation coefficient was 0.9984. The response curve of the standard peptide with the serum peptide mixture as matrix indicated that the quantitative linearity of serum MRM at the given concentrations was sufficiently valid to obtain relative quantities of target peptides.

### Example 4. Detection of target candidates in pooled serum

Before conducting individual MRM analyses using the 72 serum samples (36 healthy control group, 18 untreated HCC group, and 18 treated HCC group), a preliminary MRM analysis was performed on the target peptides/transitions using pooled serum of all patients to obtain transition information, such as the detectability in serum and the suitability of the transition. The FASTA files of the 50 proteins yielded 498 peptides and 2174 transitions on applying the hierarchy data in Skyline software. After obtaining the resulting MRM data, the final transition was selected using the following criteria: at least 2 peptides were selected per protein, and at least 3 transitions per peptide were chosen as detectable transitions that had a signal-to-noise (S/N) ratio above 3.

### Ultimately, 28 of 50 candidates met the criteria in the first screening step

### Example 5. Selection of the transitions with technical reproducibility for MRM analysis

The reproducibility of MRM analyses is critical in making quantitative measurements. In this example, the analyses were performed as follows. The pooled serum mixture from the 3 groups (the pooled serum mixture of 36 healthy control group, 18 the pooled serum mixture of untreated HCC group, and the pooled serum mixture of 18 treated HCC group) was analyzes to select the target protein that is applied to each sample from 333 transitions for 111 peptides, corresponding to the 28 candidate proteins from the first screening step.

Five repetitive scheduled MRM runs for the pooled serum mixture were performed using the retention time from the detectability experiment, using 500∼1000 transitions with window size of 120 seconds. The MRM analysis was conducted once for each sample in the order of healthy control sample, untreated sample (non-treatment), treated sample, and was repeated 5 times. The date from the MRM analysis was imported into Skyline software, exported into the peak area of each peptide transition. The peak area using the internal standard peptides was normalized prior to compare between the averaged relative quantities of each transition.

Peptides, which had low variance in intensity of normalized peptides (CV < 30%) and showed a confident difference in quantity(p-value < 0.05) between healthy control group versus untreated HCC group, and untreated HCC group versus treated HCC group, were chosen as target peptides to be applied in individual analysis. We processed MedCalc(version 12.2) statistical programs(t-TEST, ANOVA). As a result, 30 peptides, comprising 90 transitions that corresponded to 19 proteins, were selected as target protein to be applied in individual analysis

### Example 6. Selection of target marker through MRM analysis using individual serum samples

Individual MRM analysis was performed using the 90 transitions for 30 peptides selected from the second screening in Example 5, corresponding to 19 proteins. The individual sample, of which expression pattern between groups is similar to that of the pooled sample, was selected as a final target protein. The analysis was conducted once for each sample in the order of healthy control sample no.1, no. 2, untreated HCC sample no. 1, treated HCC sample no. 1 and then healthy control sample no. 3, no. 4, untreated HCC sample no. 2, treated HCC sample no. 2. The data from the MRM analysis were imported into the Skyline software, and then analyzed using MedCalc(version 12.2) statistical program.

Through this, following 9 proteins, that had similar expression patterns in the analysis of the pooled and individual samples(healthy control group, untreated HCC group, treated HCC group), were selected: long-chain specific acyl-CoA dehydrogenase, mitochondria (ACADVL), actin-binding protein, anillin (ANLN), brain acid-soluble protein 1 (BASP1), c-1-tetrahydrofolate synthase, cytoplasmic (MTHFD1), calpain-1 catalytic subunit (CAPN1), complementary C4-A (C4A), alpha-fetoprotein (AFP), filamin-B (FLNB), and polyadenylate-binding protein 1 (PABPC1).

The 9 final target proteins were verified by ROC curve. ROC curve were expressed on a two-dimensional plane to evaluate the relationship of sensitivity and specificity. The bigger AUC(area under the ROC curve) value is the better diagnosis method. The AUC values for corresponding target proteins are as follow table 2, and ROC curve and the interaction plot diagram were generated using MedCalc(version 12.2) statistical program.

**Table 2.**

| **List of proteins showing significant difference among different groups and their AUC value** | | | | | |
|---|---|---|---|---|---|
| | | | | **Area Under ROC Curve** | |
| **Numbe r** | **Protein Name** | **Gene Symbol** | **Peptide Sequence** | **Healthy control group vs. Untreated HCC group** | **Untreated HCC group vs. Treated HCC group** |
| 1 | Very long-chain specific acyl-CoA dehydrogenase, mitochondrial | ACADVL | ASNTAEVFFDGVR | 0.701 | 0.691 |
| 2 | Actin-binding protein anillin | ANLN | TQSLPVTEK | 0.920 | 0.744 |
| 3 | Brain acid soluble protein 1 | BASP1 | AEGAATEEEGTPK | 0 951 | 0.719 |
| 4 | C-1-tetrahydrofolate synthase, cytoplasmic | MTHFD1 | GVPTGFILPIR | 0.829 | 0.685 |
| 5 | Calpain-1 catalytic subunit | CAPN1 | YLGQDYEQLR | 0.946 | 0.586 |
| 6 | Complement C4-A | C4A | VGDTLNLNLR | 0.867 | 0.710 |
| 7 | Alpha- fetoprotein | AFP | GYQELLEK | 0.756 | 0.735 |
| 8 | Filamin-B | FLNB | APLNVQFNSPLPGDAVK | 0.660 | 0.707 |
| 9 | Polyadenytate-binding protein 1 | PABPC1 | EFSPFGTITSAK | 0.949 | 0.608 |

The AUC values of ACADVL, ANLN, MTHFD1, AFP, and FLNB increased in the healthy control group versus the untreated HCC group and declined in untreated HCC group versus treated HCC group. Those of BASP1, CAPN1, C4A, and PABPC1 declined in the healthy control group versus untreated HCC group and in the untreated HCC group versus treated HCC group.

In the statistical analysis, above 9 proteins were differentially expressed between healthy control group, untreated HCC group, and treated HCC group, with P-values < 0.05. The AUC values of the 9 target proteins ranged from 0.608 to 0.951 (Table 2). The AUC values of 6 proteins exceeded 0.8, those of ANLN, BASP1, CAPN1 and PABPC1 were 0.920, 0.951, 0.946 and 0.949, respectively, in the healthy control group versus untreated HCC group, reflecting the excellent specificity and sensitivity of the markers of the present disclosure.

### Example 7. Correlation of MRM analysis with Western blot

The 9 candidates from the verification step were verified. Prior to this experiment, a preliminary study was performed to confirm the abundance of these proteins in serum using PaxDb (http://pax-db.org) to determine the dilution of primary antibody (data not shown). For the Western blot, 13 of 36 healthy control group from the individual MRM analysis were selected randomly, and 13 untreated HCC group and 13 treated HCC group were analyzed. For the untreated HCC group and treated HCC group, the 13 serum samples differed from what was used in the individual MRM analysis.

To limit the variability between SDS-PAGE gels, serum from the 13 healthy control group, 13 untreated HCC group, and 13 treated HCC group were pooled and loaded onto the first lane of all SDS-PAGE gels (12%), and its intensity on each gel was used to normalize the intensities of individual samples. As a result, the bands that were generated by ACADVL and PABPC1 were inadequate to calculate intensities; thus, 7 of the 9 candidate proteins were verified by Western blot. Subsequently, the correlation between the protein quantification by MRM and Western blot was determined.

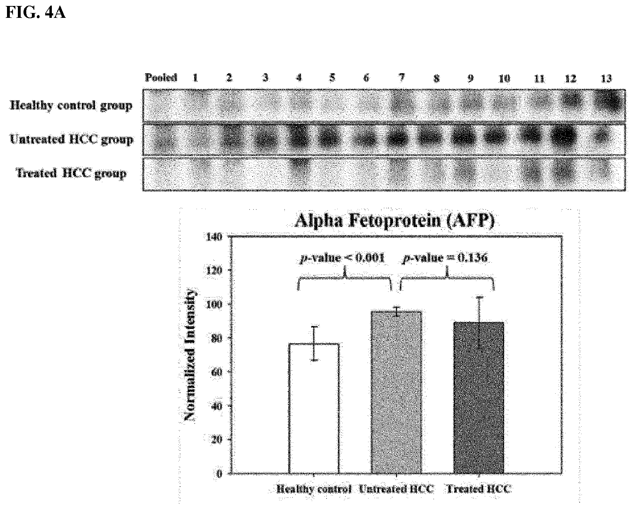

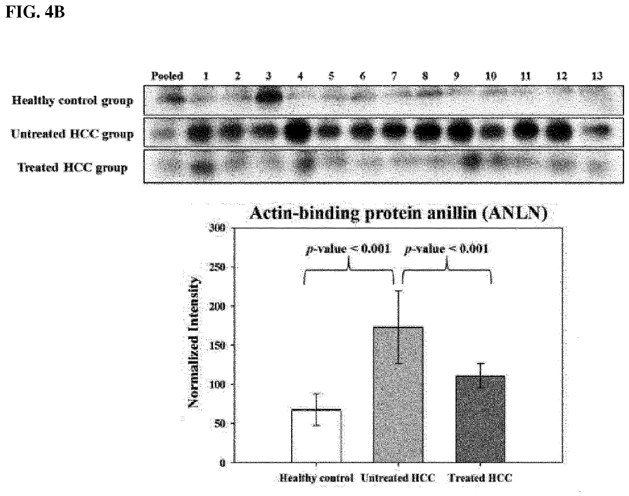

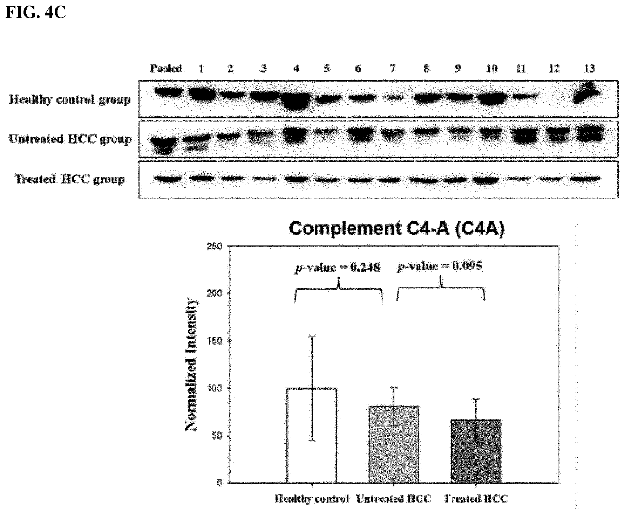

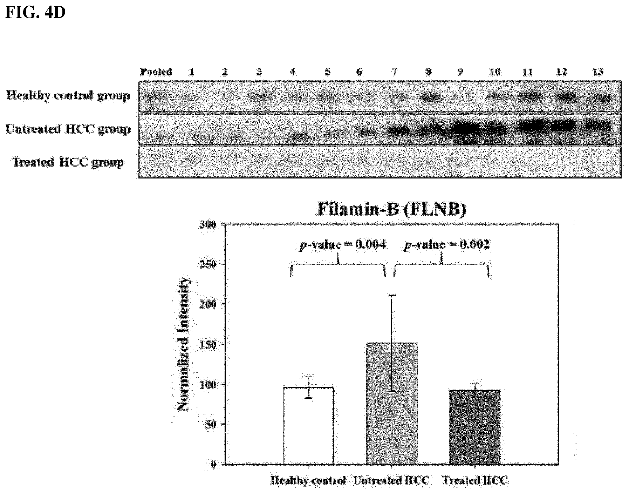

AFP, ANLN, C4A, and FLNB had the same patterns of expression by MRM analysis (Fig. 4). In the non-disease group (healthy control group, treated HCC group), AFP, ANLN, and FLNB expression decreased compared with disease group (untreated HCC group). AFP is an established serum biomarker for HCC and was verified to have consistent expression by MRM and Western blot. C4A declined in the healthy control group versus untreated HCC group and in untreated HCC group versus treated HCC group. In the comparison between MRM- and antibody-based verification, the relative quantities of 4 proteins (AFP, ANLN, FLNB, and C4A) were similar. In contrast, by Western blot, BASP1, MTHFD1, and CAPN1 had disparate expression patterns compared with MRM analysis.

### Example 8. Combination and analysis of the multimarker panel

It has been found that a multiprotein panel provide an improved discriminatory power over single markers.

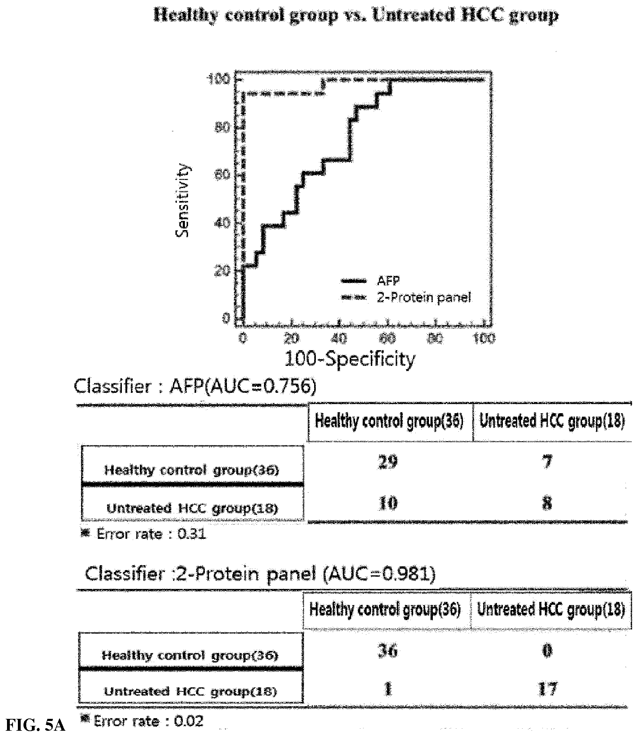

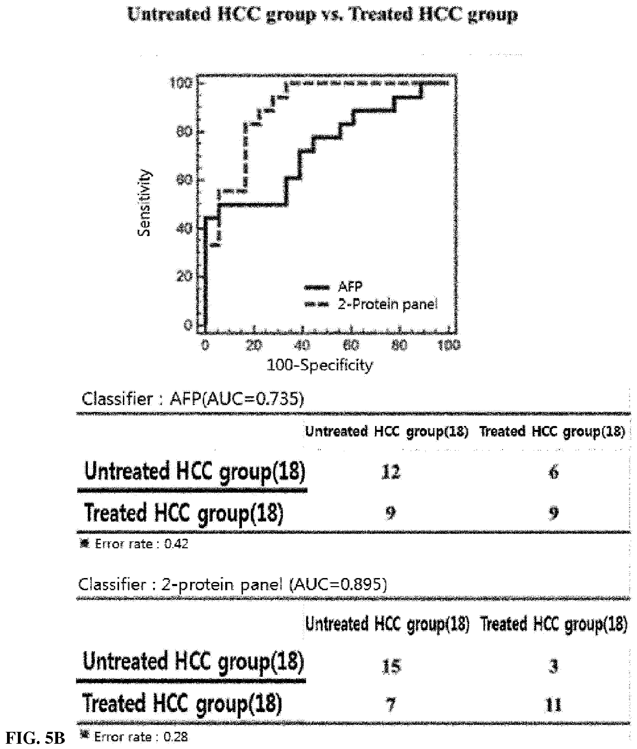

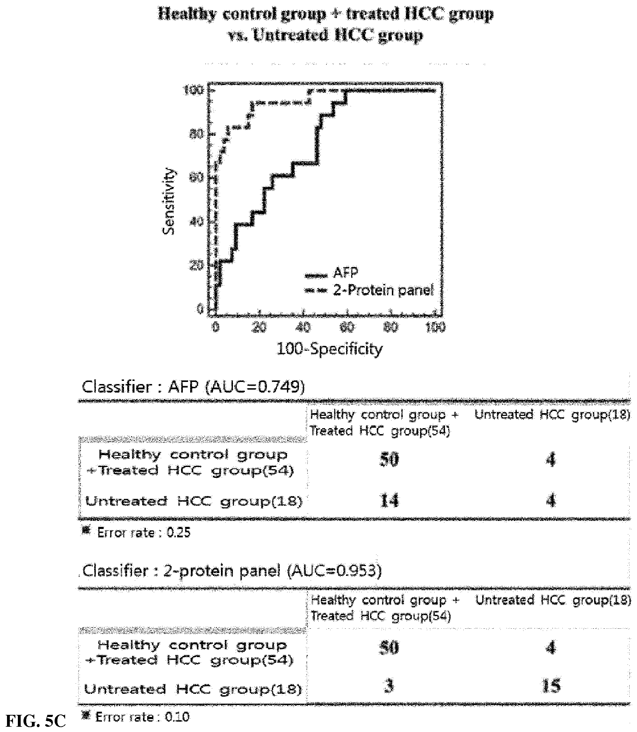

Four proteins that were validated by MRM analysis and Western blot were used to generate the multimarker panel. AFP and C4A showed colinearity, with a variance inflation factor (VIF) above 10; thus, these proteins were excluded in the final multi-marker model. The ultimate model comprised ANLN and FLNB, for which AFP was the control model as a single marker. Three types of comparisons were made: healthy control group versus untreated HCC group, untreated HCC group versus treated HCC group, and healthy control group plus treated HCC group versus untreated HCC group (Figs. 5A, 5B, and 5C).

When AFP was used as the lone classifier, 8 of 18 untreated HCC group and 29 of 36 healthy control group were classified correctly, demonstrating an AUC of 0.756 and 31% error rate.

In contrast, when the 2-marker panel of the present disclosure was used, 17 of 18 untreated HCC group and all 36 healthy control group were classified correctly, with an AUC of 0.981 and 2% error rate, indicating that the 2-marker panel had improved discriminatory power compared with the AFP model (Fig. 5A).

As shown in Fig 5B, the AFP-only model classified 12 of 18 untreated HCC group and 9 of 18 treated HCC group correctly (AUC = 0.735, error rate = 42%), whereas the 2-marker panel of the present disclosure separated 15 of 18 untreated HCC group and 11 of 18 treated HCC group successfully (AUC=0.895, error rate=28%). Thus, in identifying patients in the untreated HCC group versus treated HCC group, the discriminatory power of the 2-marker panel was outstanding compared with AFP alone.

The AFP-only model classified 4 of 18 disease group (untreated HCC group) and 50 of 54 nondisease group (healthy control group, treated HCC group), with an AUC of 0.749 and error rate of 25% (Fig. 5C). Whereas the 2-marker panel identified 15 of 18 disease group and 50 of 54 nondisease group (AUC=0.953, error rate=10%), outstanding compared with AFP alone.

These results demonstrate that in all comparisons, the 2-marker panel of the present disclosure has greater discriminatory power compared to the traditional single marker AFP.

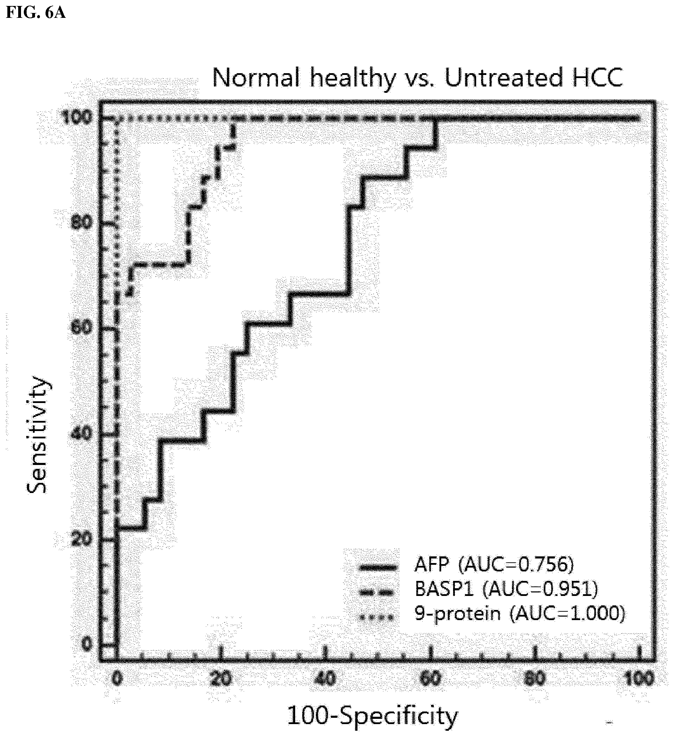

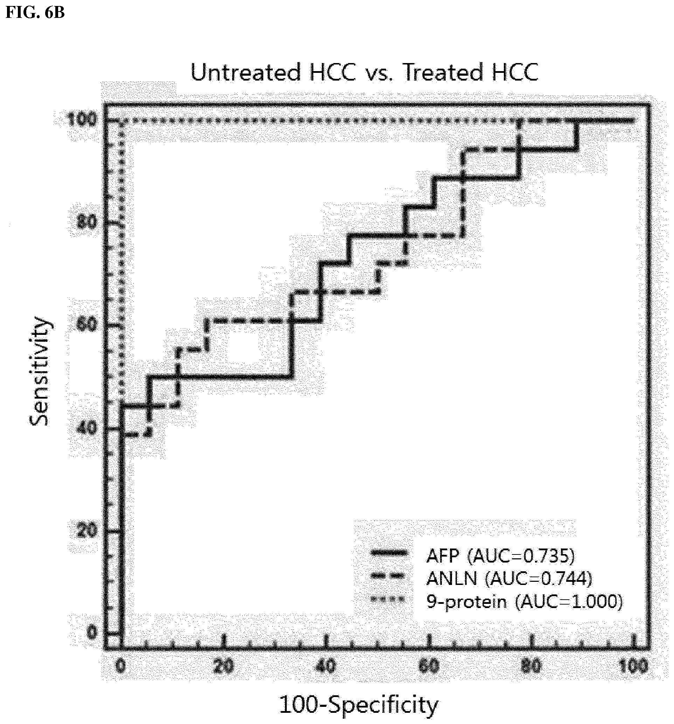

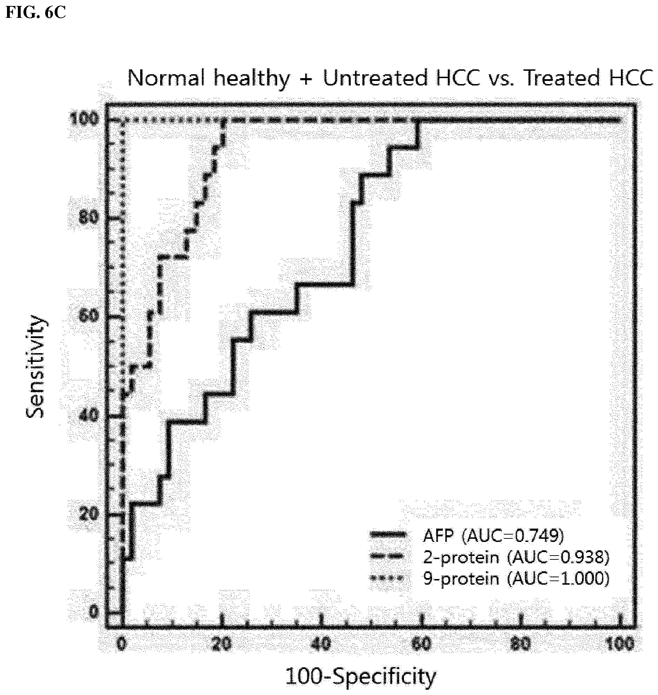

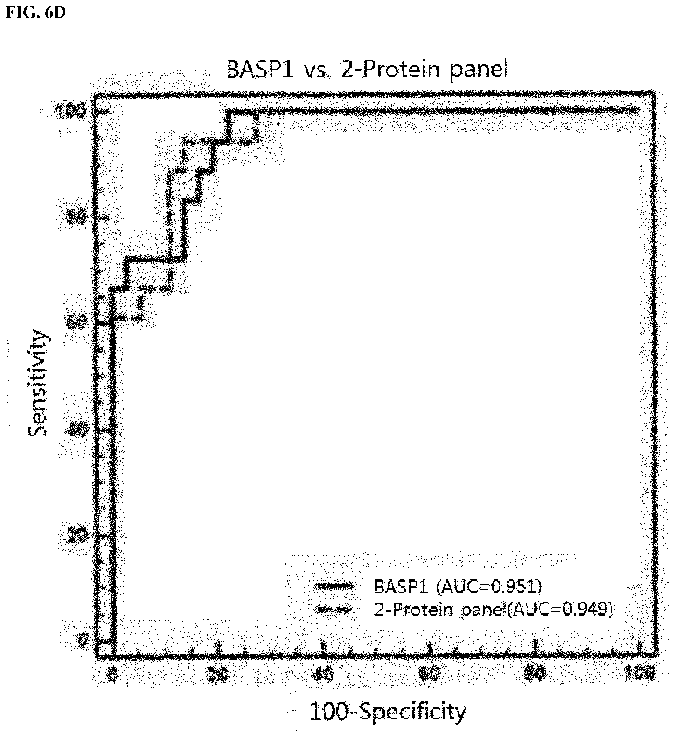

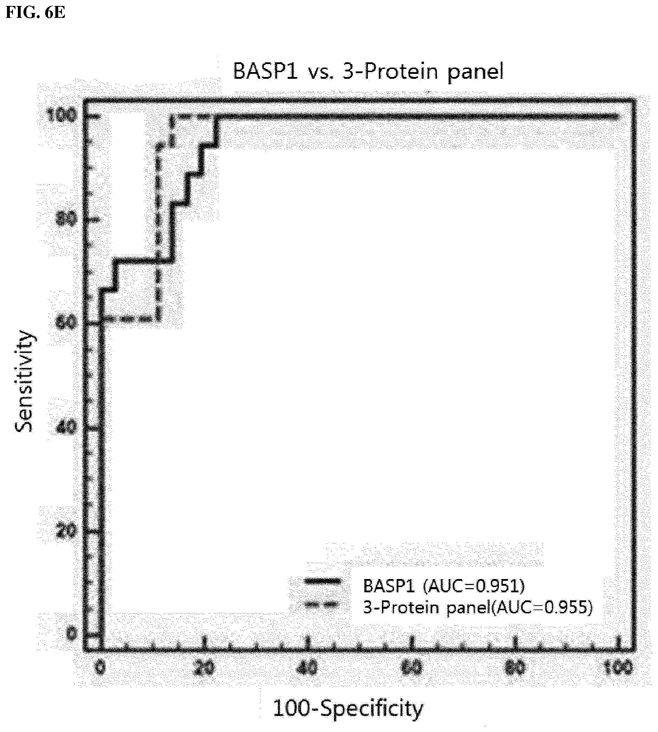

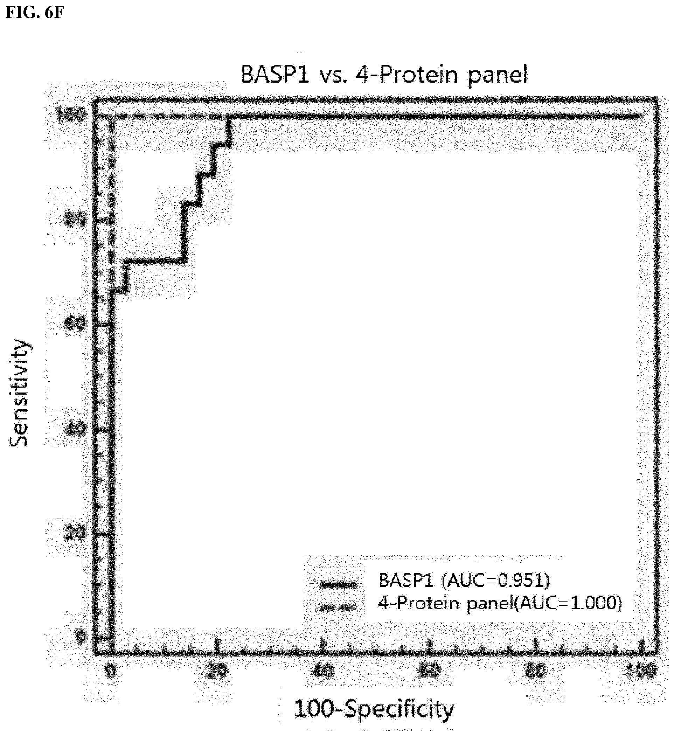

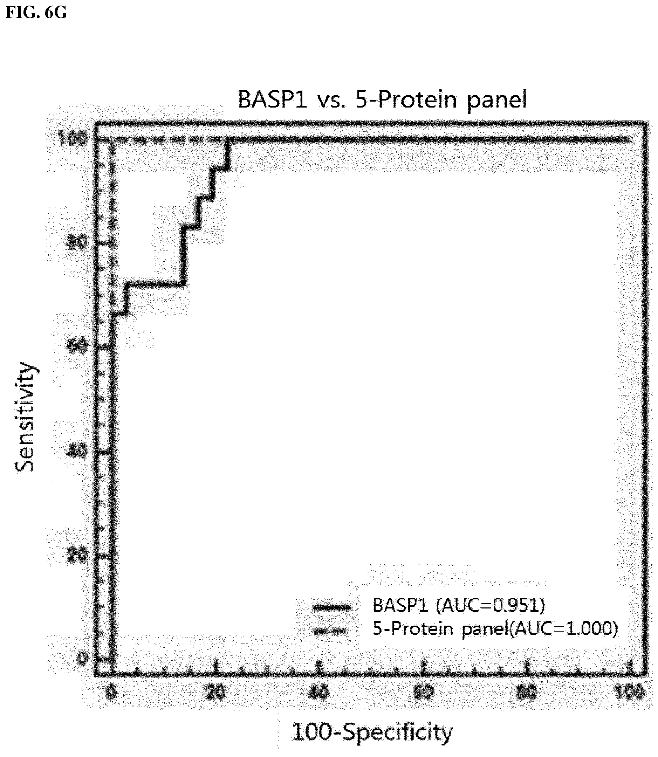

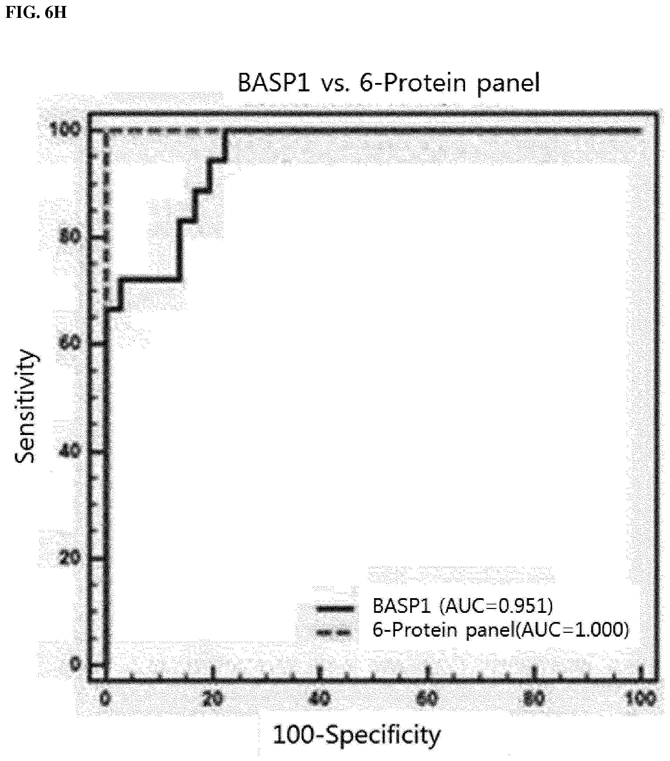

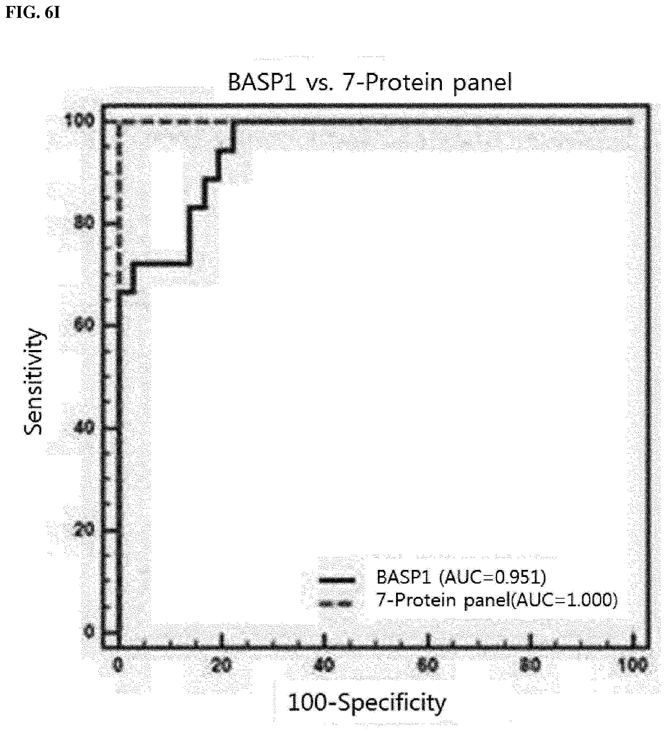

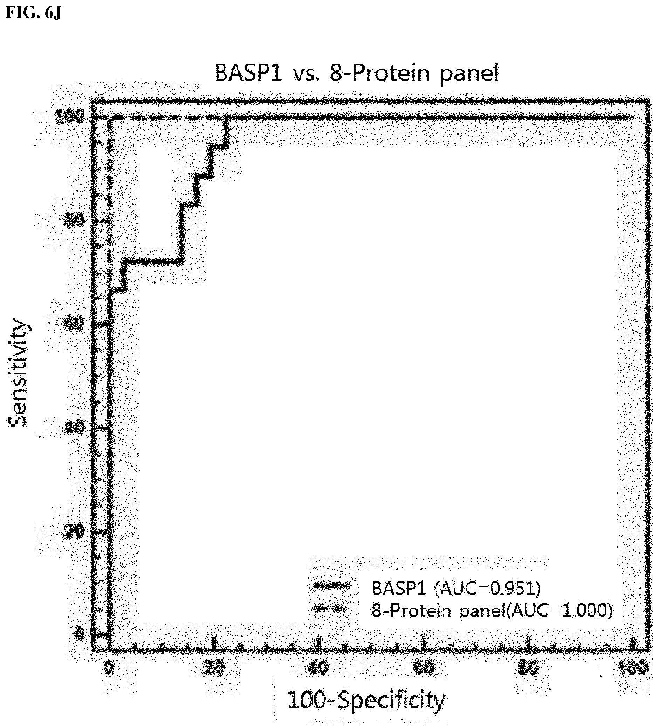

Also as shown in Fig 6a to j, the various other liver cancer marker panels combining several individual markers were generated by performing Logistic regression (LR) analysis,. The markers BASP1 and ANLN having the highest AUC value and AFP, and 9 markers (ACADVL, ANLN, BASP1, MTHFD1, CAPN1, C4A, AFP, FLNB, and PABPC1) were combined and these combinations generated a multiprotein panel. We analyzed these combinations to discriminate liver cancer.

By Logistic regression statistical analysis, 9-protein panel, which comprised 9 proteins, was constructed. This panel had improved discriminatory power compared with the AFP panel in identifying patients in the untreated HCC group versus treated HCC group, treated HCC group versus normal healthy control group, and untreated HCC group versus normal healthy control group successfully(AUC = 1.00).

Further 2-8 protein marker panels, which comprised one or more marker proteins selected from 8 marker proteins except AFP, were constructed according to the criteria of ROC curve of BASP1 of which AUC was the highest among markers. And we constructed the ROC curve and compared the AUC value of untreated HCC group with that of normal healthy control group.

As a result, the AUC value of 2-protein panel was 0.949, that of 3-protein panel was 0.955, and that of 4,5,6,7,8-protein panel were all 1.000. So, two or more combination panel as well as one-panel had outstanding specificity and sensitivity.

The various singular/plural permutations may be expressly set forth herein for sake of clarity.

Unless defined or interpreted otherwise, all technical and scientific terms and any acronyms used herein have the same meanings as commonly understood by one of ordinary skill in the art in the field of the invention.

## Claims

1. A method for diagnosing or prognosing heptacellular carcinoma (HCC) comprising:
detecting the concentration of long-chain specific acyl-CoA dehydrogenase (ACADVL), and actin-binding protein, anillin (ANLN), and brain acid-soluble protein 1 (BASP1) at a nucleic acid and/or protein level in a sample from a subject in need thereof; and
comparing the detection result to that of a corresponding marker from a control sample wherein the change in the concentration of the biomarker at a nucleic acid and/or protein level in the subject from the control sample indicates the presence of HCC in the subject, wherein the change is the increased concentration of the biomarkers ACADVL, and ANLN and the decreased concentration of biomarker BASP1 in the subject compared to the control.

2. The method of claim 1, wherein the detection is performed by at least one of a microarray, a gene amplification, an antigen-antibody reaction of a mass spectrometry.

3. The method of claim 1, wherein the detection of the concentration of the biomarkers at the nucleic acid level is performed by at least one of a polymerase chain reaction, a reverse transcription polymerase chain reaction, a competitive polymerase chain reaction, a RNase or S1 nuclease assay, an in situ hybridization, a DNA microarray or a norther blot assay.

4. The method of claim 1, wherein the detection of the concentration of the biomarkers at the protein level is performed by at least one of a western blot, an ELISA, an radioimmunoassay, an immunodiffusion assay, and immunoelectrophoresis, an immunostaining, an immunoprecipitation, a complement fixation assay, a FACS, an mass spectrometry, or a protein microarray.

5. The method of claim 1, biomarkers further comprise:
polyadenylate-binding protein 1 (PABPC1), calpain-1 catalytic subunit (CAPN1), complementary C4-A (C4A), and c-1-tetrahydrofolate synthase, cytoplasmic (MTHFD1); or
PABPC1, CAPN1, C4A, MTHFD1, and filamin-B (FLNB).

6. The method of claim 1, further comprising detecting the concentration of a biomarker alpha fetoprotein (AFP).

7. Use of the biomarkers selected from the group consisting ACADVL, and ANLN, and BASP1 for diagnosing or prognosis of HCC according to the method of any one of claim 1 to 6.

8. The Use of claim 7, biomarkers further comprise:
PABPC1, CAPN1, C4A, and MTHFD1; or
PABPC1, CAPN1, C4A, MTHFD1 and FLNB.

9. Use of a kit for diagnosing or prognosis of HCC according to the method of any one of claims 1 to 6, the kit comprising an agent to detect the concentration of ACADVL, and ANLN, and BASP1 at a nucleic acid and/or protein level.

10. Use of the kit of claim 9, wherein the biomarkers further comprise:
PABPC1, CAPN1, C4A, and MTHFD1; or
PABPC1, CAPN1, C4A, MTHFD1 and FLNB.

11. Use of the kit of claim 9, wherein the agent is a monoclonal antibody, a polyclonal antibody, a substrate, an aptamer, an avimer, a peptidomimetic, a receptor, a ligand or a cofactor.

12. Use of the
Kit of claim 9, wherein the kit is formulated as a ELISA kit, a dip stick rapid kit, a MRM analysis kit, a microarray kit, a nucleic acid amplification kit, or an immunoassay kit.

13. Use of the kit of claim 9, further comprising a biomarker AFP.

## Patentansprüche

1. Verfahren zum Diagnostizieren oder Prognostizieren eines Leberkarzinoms (HCC), das Folgendes umfasst:
Detektieren der Konzentrationen langkettiger spezifischer Acyl-CoA-Dehydrogenase (ACADVL) und Actin-bindenden Proteins, Anilins (ANLN) und gehirnsäurelöslichen Proteins 1 (BASP1) auf einer Nukleinsäure- und/oder Proteinebene in einer Probe von einem behandlungsbedürftigen Probanden; und
Vergleichen des Detektionsergebnisses mit dem eines entsprechenden Markers aus einer Kontrollprobe, wobei die Änderung der Konzentration des Biomarkers auf einer Nukleinsäure- und/oder Proteinebene in dem Probanden von der Kontrollprobe das Vorliegen eines HCC in dem Probanden anzeigt, wobei die Änderung die erhöhte Konzentration der Biomarker ACADVL und ANLN und die verringerte Konzentration des Biomarkers BASP1 in dem Probanden im Vergleich zu der Kontrolle ist.

2. Verfahren nach Anspruch 1, wobei die Detektion von einem Mikroarray und/oder einer Genamplifikation und/oder einer Antigen-/Antikörperreaktion von einer Massenspektrometrie durchgeführt wird.

3. Verfahren nach Anspruch 1, wobei die Detektion der Konzentration der Biomarker auf der Nukleinsäureebene durch eine Polymerasekettenreaktion und/oder eine umgekehrte Transkriptionspolymerasekettenreaktion und/oder eine kompetitive Polymerasekettenreaktion und/oder ein RNase-Assay und/oder Sl-Nuklease-Assay und/oder eine In-situ-Hybridisierung und/oder ein DNA-Mikroarray und/oder ein Northern-Blot-Assay durchgeführt wird.

4. Verfahren nach Anspruch 1, wobei die Detektion der Konzentration der Biomarker auf der Proteinebene durch ein Western-Blot und/oder ein ELISA und/oder ein Radioimmun-Assay und/oder ein Immundiffusions-Assay und/oder eine Immunelektrophorese und/oder eine Immunfärbung und/oder eine Immunpräzipitation und/oder ein Komplementbindungs-Assay und/oder ein FACS und/oder eine Massenspektrometrie und/oder ein Protein-Mikroarray durchgeführt wird.

5. Verfahren nach Anspruch 1, wobei die Biomarker ferner Folgendes umfassen:
Polyadenylat-Bindungsprotein 1 (PABPC1), Calpain-l-katalytische Untereinheit (CAPN1), komplementäres C4-A (C4A) und zytoplasmische c-1-Tetrahydrofolatsynthase (MTHFD1); oder
PABPC1, CAPN1, C4A, MTHFD1 und Filamin-B (FLNB).

6. Verfahren nach Anspruch 1, das ferner das Detektieren der Konzentration eines Alphafetoprotein-Biomarkers (AFP) umfasst.

7. Verwendung der Biomarker, die aus der Gruppe gewählt sind, die aus ACADVL, ANLN und BASP1 besteht, zum Diagnostizieren oder Prognostizieren von HCC entsprechend dem Verfahren nach einem der Ansprüche 1 bis 6.

8. Verwendung von Anspruch 7, wobei die Biomarker ferner Folgendes umfassen:
PABPC1, CAPN1, C4A und MTHFD1; oder
PABPC1, CAPN1, C4A, MTHFD1 und FLNB.

9. Verwendung eines Kits zum Diagnostizieren oder Prognostizieren von HCC entsprechend dem Verfahren nach einem der Ansprüche 1 bis 6, wobei das Kit einen Wirkstoff zum Detektieren der Konzentration von ACADVL, ANLN und BASP1 auf einer Nukleinsäure- und/oder Proteinebene umfasst.

10. Verwendung des Kits nach Anspruch 9, wobei die Biomarker ferner Folgendes umfassen:
PABPC1, CAPN1, C4A und MTHFD1; oder
PABPC1, CAPN1, C4A, MTHFD1 und FLNB.

11. Verwendung des Kits nach Anspruch 9, wobei der Wirkstoff ein monoklonaler Antikörper, ein polyklonaler Antikörper, ein Substrat, ein Aptamer, ein Avimer, ein Peptidomimetikum, ein Rezeptor, ein Ligand oder ein Cofaktor ist.

12. Verwendung des Kits nach Anspruch 9, wobei das Kit als ein ELISA-Kit, ein Messstabschnellkit, ein MRM-Analysekit, ein Mikroarraykit, ein Nukleinsäureverstärkungskit oder ein Immunassaykit ausgearbeitet ist.

13. Verwendung des Kits nach Anspruch 9, das ferner einen AFP-Biomarker umfasst.

## Revendications

1. Procédé de diagnostic ou de pronostic d'un carcinome hépatocellulaire (HCC) comprenant :
la détection de la concentration en acyl-CoA déshydrogénase spécifique à longue chaîne (ACADVL), et une protéine liant l'actine, l'anilline (ANLN) et une protéine cérébrale soluble dans l'acide 1 (BASP1) au niveau de l'acide nucléique et/ou des protéines dans un échantillon d'un sujet le nécessitant ; et
la comparaison du résultat de détection à celui d'un marqueur correspondant d'un échantillon témoin dans lequel la modification de la concentration du biomarqueur au niveau de l'acide nucléique et/ou des protéines chez le sujet de l'échantillon témoin indique la présence d'un HCC chez le sujet, dans lequel la modification est l'augmentation de la concentration des biomarqueurs ACADVL, et ANLN et la diminution de la concentration du biomarqueur BASP1 chez le sujet comparé au témoin.

2. Procédé selon la revendication 1, dans lequel la détection est réalisée par au moins l'un d'un microréseau, d'une amplification génique, d'une réaction antigène-anticorps d'une spectrométrie de masse.

3. Procédé selon la revendication 1, dans lequel la détection de la concentration des biomarqueurs au niveau d'acide nucléique est réalisée par au moins l'une d'une réaction d'amplification en chaîne par polymérase, d'une réaction d'amplification en chaîne par polymérase - transcriptase inverse, d'une réaction d'amplification en chaîne par polymérase compétitive, d'un dosage de la RNase ou de la nucléase S1, d'une hybridation in situ, d'un microréseau d'ADN, d'un dosage par transfert de type Northern.

4. Procédé selon la revendication 1, dans lequel la détection de la concentration des biomarqueurs au niveau des protéines est réalisée par au moins l'un d'un transfert de type Western, d'un ELISA, d'un dosage radioimmunologique, d'un dosage par immunodiffusion, d'une immunoélectrophorèse, d'une immunocoloration, d'une immunoprécipitation, d'un test de fixation du complément, d'un FACS, d'une spectrométrie de masse, ou d'un microréseau de protéine.

5. Procédé selon la revendication 1, les biomarqueurs comprennent en outre :
la protéine de liaison du polyadénylate 1 (PABPC1), la sous-unité catalytique de la calpaïne-1 (CAPN1), le CA-4 complémentaire (C4A), et la c-1-tétrahydrofolate synthase, cytoplasmique (MTHFD1) ; ou
PABPC1, CAPN1, C4A, MTHFD1, et la filamine B (FLNB).

6. Procédé selon la revendication 1, comprenant en outre la détection de la concentration d'un biomarqueur alpha-foetoprotéine (AFP).

7. Utilisation des biomarqueurs sélectionnés dans le groupe constitué d'ACADVL, et ANLN, et BAPS1 pour le diagnostic ou le pronostic d'un HCC conformément au procédé selon l'une quelconque des revendications 1 à 6.

8. Utilisation selon la revendication 7, les biomarqueurs comprennent en outre :
PABPC1, CAPN1, C4A et MTHFD1 ; ou
PABPC1, CAPN1, C4A, MTHFD1 et FLNB.

9. Utilisation d'un kit pour le diagnostic ou le pronostic d'un HCC conformément au procédé selon l'une quelconque des revendications 1 à 6, le kit comprenant un agent pour détecter la concentration en ACADVL, et en ANLN, et en BASP1 au niveau de l'acide nucléique et/ou des protéines.

10. Utilisation du kit selon la revendication 9, dans laquelle les biomarqueurs comprennent en outre :
PABPC1, CAPN1, C4A et MTHFD1 ; ou
PABPC1, CAPN1, C4A, MTHFD1 et FLNB.

11. Utilisation du kit selon la revendication 9, dans laquelle l'agent est un anticorps monoclonal, un anticorps polyclonal, un substrat, un aptamère, un avimère, un peptidomimétique, un récepteur, un ligand ou un cofacteur.

12. Utilisation du kit selon la revendication 9, dans laquelle le kit est formulé sous la forme d'un kit ELISA, d'un kit rapide sur bandelette, d'un kit d'analyse MRM, d'un kit de microréseau, d'un kit d'amplification d'acide nucléique, ou d'un kit de dosage immunologique.

13. Utilisation du kit selon la revendication 9, comprenant en outre un biomarqueur AFP.
